# EUROPEAN PATENT APPLICATION

(11) **EP 1 466 610 A1**
(43) Date of publication of application: **13.10.2004**
(21) Application number: 02783628.7
(22) Date of filing: 26.11.2002
(51) Int. Cl.: A61K 38/00, A61K 9/14, A61K 38/22, A61K 38/26, A61K 38/28, A61K 47/02, A61K 47/12, A61K 47/34, A61K 47/36, A61K 47/38

(54) **MEDICINAL COMPOSITIONS FOR NASAL ABSORPTION**

(30) Priority: 26.11.2001 JP 2001359559
(71) Applicant: Daiichi Suntory Pharma Co., Ltd., Tokyo 102-8530 (JP); DOTT RESEARCH LABORATORY, Yokohama-shi, Kanagawa 224 (JP)
(72) Inventor: MINAMITAKE, Yoshiharu, Nitta-gun, Gunma 370-0311 (JP); TSUKADA, Yoshio, Tatebayashi-shi, Gunma 374-0075 (JP); KANAI, Yasushi, Ashikaga-shi, Tochigi 326-0824 (JP); YANAGAWA, Akira, Yokohama-shi, Kanagawa 224-0051 (JP)
(74) Representative: Hiebl, Inge Elisabeth
(86) International application number: PCT/JP2002/012337
(87) International publication number: WO 2003/045418

(57) **Abstract**

A pharmaceutical composition for nasal administration exhibits an improved bioavailability of a biologically active polypeptide, the active ingredient of the pharmaceutical composition. Specifically, the composition is prepared by uniformly dispersing and embedding a biologically active acidic polypeptide having an isoelectric point of 7 or lower on the surfaces of a polyvalent metal compound carrier with the help of an additive capable of dispersing and embedding the polypeptide on the surfaces of the carrier. The polyvalent metal compound carrier is a metal compound with a valent of 2 or higher that is either insoluble or little soluble in water, examples being aluminum compounds, calcium compounds, magnesium compounds, silicone compounds, iron compounds, and zinc compounds.

## Description

### TECHNICAL FIELD

The present invention relates to a pharmaceutical composition for nasal absorption and, more particularly, to a pharmaceutical composition for nasal absorption that contains as an active ingredient a biologically active acidic polypeptide with an isoelectric point of 7 or lower and additives that enhance the bioavailability of the peptide.

### TECHNICAL BACKGROUND

Biologically active polypeptides are high-molecular weight compounds exhibiting various specific pharmacological activities and are compounds of significant usefulness that have been used in medical fields for various purposes. For example, glucagon-like peptide I (referred to as GLP-1, hereinafter), a peptide hormone derived from glucagon precursor (proglucagon), is known (Bell *et al., Nature, 304, 368, 1983*). Proglucagon that has been identified in mammals is a precursor protein consisting of 160 amino acids and is produced in pancreatic islet (islets of Langerhans) A-cells and intestinal L-cells. In pancreas, proglucagon is processed by a processing enzyme into glucagon and a major proglucagon fragment whereas, in intestine, proglucagon is processed in a different manner to produce glicentin, GLP-1, and glucagon-like peptide-2 (referred to as GLP-2, hereinafter) (Mojsovr *et al., J. Biol*. *Chem., 261, 11880, 1986*).

The peptide formed of amino acids 72-108 of proglucagon (equivalent to GLP-1) exhibits an activity to promote insulin secretion. Also, GLP-1(7-37), which forms as a result of removal of the first 6 N-terminal amino acids from GLP-1, and GLP-1(7-36)NH₂, which forms as a result of amidation of GLP-1(7-37) at position 36, are the most effective promoters of insulin secretion among other previously known promotors of insulin secretion (Mojsov *et al., J. Clin. Invest., 79, 616, 1987*). They also have an activity to suppress glucagon secretion. It has been shown that GLP-1 is secreted from intestinal L-cells into blood circulation in the form of GLP-1(7-36)NH₂ (Gutniak *et al*., *N. Engl. J. Med., 326, 1316*, *1993*).

GLP-1 (7-36)NH₂ is immediately secreted from intestinal L-cells in response to stimuli of food ingestion and acts on pancreas to promote secretion of insulin. At the same time, it acts to decrease glucagon secretion, increase mRNA expression in insulin-secreting cells, decrease gluconeogenesis in liver, and suppresses activity of gastrointestinal tract. This suggests a vital role of GLP-1, including GLP-1(7-36)NH₂, as an incretin (stimulant of insulin secretion) that is tailored to the requirements of energy metabolism in a human body.

Having the above-described biologically activities, the peptides find an application as a medication for treating diabetes. Specifically, GLP-1(7-36)NH₂ can be administered before meal to suppress the post-meal rise in the blood glucose level so that it can act as an effective therapeutic agent for type II diabetes patients. Sulfonyl urea drugs, which have a similar activity of promoting insulin secretion, are associated with the risk of an excessively low blood glucose level since the drug exhibits its activity irrespective of the blood glucose level. When administered over a long-term period, the drugs can also cause the insulin-producing cells to become less active. In contrast, since the activity of GLP-1(7-36)NH₂ to promote insulin secretion is regulated by a feedback mechanism reflecting the blood glucose concentration, GLP-1(7-36)NH₂ rarely brings about excessively low blood glucose level. Further, GLP-1(7-36)NH₂ stimulates the insulin-producing cells. Thus, a sharp contrast exists between GLP-1(7-36)NH₂ and the sulfonyl urea drugs presently in clinical use as a medication for diabetes.

The various activities of GLP-1(7-36)NH₂, including suppression of gluconeogenesis in liver, activation of insulin-producing cells, promotion of sugar intake by muscle, suppression of activity of gastrointestinal tract, and appetite suppression through central nervous system, have led to an expectation that, aside from its activity to correct the post-meal blood glucose levels, administration of GLP-1(7-36)NH₂ over a long term period can normalize and activate the integrity of systemic glucose metabolism, and suppress obesity, one of the major factors of diabetes.

Another peptide that has a similar incretin activity *(i.e.*, stimulation of insulin secretion) to GLP-1(7-36)NH₂ is exendin-4, which was isolated from the Gila monster (a species of reptile) and the structure of which was determined. The peptide is less susceptible to degradation in blood plasma than GLP-1(7-36)NH₂ and is therefore capable of retaining the activity to promote insulin secretion for a prolonged period of time. As with GLP-1(7-36)NH₂, exendin-4 is known to induce differentiation/neogenesis of β-cells.

Because 8th position of GLP-1(7-37) and GLP-1(7-36)NH₂ is cleaved by dipeptidyl peptidase IV (DPP-IV) existing within living body, derivative which Ala in 8th position is substituted with an amino acid not easily cleaved such as Val, i.e., [Val⁸]-GLP-1(7-37), and derivative in which GLP-1 is modified with fatty acid to delay the apparent plasma half-life by dissolving rate controlling, i.e., [Lys²⁶, ε-NH{γ-Glu(N-α-palmitoyl)}]- GLP-1(7-37) are hoped to have the same effect. On the other hand, recent study showed that GLP-1(9-37) which is cleaved at 8th position have the same blood glucose level decreasing effect (Deacon et al., *Am. J. Physiol. Endocrinol. Matb. 282, 873-879, 2002)*. Due to this finding, it is necessary to verify the advantage of the conversion of amino acid residue of GLP-1(7-36)NH₂ at 8th position, and the modification of GLP-1(7-36)NH₂ with fatty acid to delay the apparent plasma half-life.

Gastric inhibitory polypeptide (GIP, hereinafter), a peptide that stimulates insulin secretion glucose-dependently, is distinguished from GLP-1 and exendin-4 in that the peptide, in addition to its activity to promote insulin secretion, can promote secretion of glucagon.

Of these peptide incretins, GLP-1(7-36)NH₂ has an amino acid sequence common among mammals and is considered an ideal medicament against diabetes. However, GLP-1(7-36)NH₂ is hardly absorbed from gastrointestinal tracts due to its nature as peptide. This significantly hinders the development of the peptide as a medicament for diabetes.

As is known, aside from oral administration, the peptide may be administered percutaneously by subcutaneous injection. However, long-term subcutaneous injection must be controlled under observation of medical doctors, and considering the anibulant burden and pains in injection sites, injection is not a suitable route for long-term administration of the medicament for treating diabetes. Though the peptide can effectively correct high blood glucose levels when administered after each meal, subcutaneous injection given 3 times a day is apparently not a practical way to administer the peptide. Further, it is not likely that self-controlled type insulin injection which is administered not only to type I diabetes patient, but also to type II diabetes patient would be used in combination with self-controlled type GLP-1 injectable formulation.

In order to solve the above-described problems, attempts have been made to allow GLP-1 (7-36)NH₂ to be absorbed through mucosa of oral cavity in the form of a patch preparation (Gutniak *et al., Diabetes care, 20, 1874, 1997*). This particular form of administration, however, involves the use of an absorption enhancers, in this example, sodium taurocholate, which is a type of bile acid and is highly irritant. As a result, irritancy is unavoidable and mucosa may be lost, making this administration route unsuitable for long-term administration.

Thus, to this date there has been no practical non-injection technique for administering GLP-1 (7-36) NH₂ and other peptide incretins that is safe, can achieve a high bioavailability, and is suitable for frequent delivery of the drug. The development of such a technique has been longed for.

Unlike low-molecular weight compounds, biologically active polypeptides are not effectively administered by any administration route but injection. The principal reasons for this are that biologically active polypeptides are subjected to digestion by digestive enzymes present in stomach, and small and large intestines or in absorptive epithelia of these organs, nasal cavity, and lungs and that the polypeptides, due to their large molecular weights, are not transported through typical transportation pathways. For these reasons, nasal peptide preparations intended for nasal absorption have recently been proposed to serve as a viable non-injection technique for administering peptides. Typically, such a nasal peptide preparation is administered by spraying a peptide solution with a nebulizer into a nasal cavity in the presence of absorption enhancers. One problem with this approach is that, of various known biologically active polypeptides, certain peptides, including GLP-1(7-36)NH₂, that have an isoelectric point (pI, hereinafter) in an acidic or neutral range tend to become unstable in an acidic or neutral solution.

For example, observations by the present inventors have revealed that, while a solution of GLP-1(7-36)NH₂, when nasally administered to rats and other animals, is absorbed to some extent, the peptide becomes insoluble when the solution is stored over several tens of hours (see Reference Example in the following). Thus, the solution preparation is not suitable for use with the pharmaceutical composition even if it is of the type in which the peptide is dissolved each time the preparation is used.

Likewise, glucagon and insulin have isoelectric point in an acidic or neutral pH range and is known to become insoluble or crystallize in an acidic or neutral solution. Many such peptides are known that have an isoelectric point in an acidic or neutral pH range and thus become insoluble or crystallize in an acidic or neutral solution. Therefore, it is substantially impossible to nasally administer these peptides in the form of an acidic or neutral solution preparation.

On the other hand, it is expected that acidic biologically active polypeptides are highly soluble in an alkaline (basic) solvent. However, when acidic biologically active polypeptides were exposed to a basic solution, the acidic biologically active polypeptide not only becomes susceptible to degradation such as hydrolysis, which can also take place in an acidic environment, but also tends to undergo racemization. As a result, its chemical stability is decreased. Both of acidic and basic biologically active polypeptides can undergo these side reactions. It is known, for example, that casein (pI = about 4.6), a basic biologically active polypeptide, becomes unstable as its amino acid residues of aspartic acid, phenylalanine, glutamic acid, and alanine undergo racemization in a basic solution (Friedman *et al., J. Food Sci*., *47*, *760-764, 1982*). Organic acids include a variety of materials, including acetic acid and butyric acid, each being a biological compound, and long-chain carboxylic acids such as octanoic acid and decanoic acid both of were neutrients. Many of these organic acids can be used as additives in pharmaceutical composition. On the other hand, many of organic bases, such as serotonin and dopamine, are known to exhibit pharmacological activities. Alkaline metals such as sodium are in many cases not suitable for use as an additive in a pharmaceutical composition since they often make it difficult to adjust pH of the composition and tend to form a salt with an acidic peptide, thus affecting the properties of the peptide. For these reasons, it is not preferred, considering the chemical stability and the choices of the additive components, to provide the acidic biologically active polypeptide in the form of an alkaline solution.

As described above, it is not preferred to provide acidic biologically active polypeptides in the form of an acidic or neutral solution preparation nor in the form of an alkaline solution preparation. Thus, acidic biologically active polypeptides are not suitable for use in the solution preparations for nasal administration.

For certain types of biologically active polypeptides, such as insulin, calcitonin, parathyroid hormone (PTH), human growth hormone (HGH), and hypothalamus hormone (LH-RH), powder preparations for nasal absorption have been proposed as an alternative to the solution-type nasal preparation. Many compounds have been examined for the potential as a carrier for these powder preparations for nasal administration, and different powder compositions using several different carriers have been proposed thus far for nasal administration of the biologically active polypeptides.

Through the course of studies, it has been found that substances that are insoluble or little soluble in water but can dissolve in water under acidic conditions can serve as a highly effective carrier for the powder preparation for nasal administration of biologically active polypeptides. For example, pharmaceutical compositions for nasal administration have been proposed that uses as a carrier a polyvalent metal compound, such as hydroxyapatite and calcium carbonate (Japanese Patent Laid-Open Publication No. Hei 8-27031), substances having the ability to repair or protect mucosa, in particular gastric mucosa (Japanese Patent Laid-Open Publication No. Hei 9-255586), or powdered grains (Japanese Patent Laid-Open Publication No. 2000-239187).

However, one powder preparation, which was prepared by dispersing and adsorbing GLP-1(7-36)NH₂ onto the carrier such as the polyvalent metal compound, showed bioavailability of GLP-1(7-36)NH₂ of about 4% in dogs and 1% or less in monkeys when nasally administered to the animals. Thus, the nasal absorption of the preparation was less than satisfactory.

The biologically active polypeptides, such as GLP-1(7-36)NH₂, whose isoelectric points are in an acidic or neutral pH range have a low solubility in an acidic or neutral pH range and, even when dissolved in a solution, tend to aggregate. These polypeptides cannot achieve a sufficient bioavailability not only when nasally administered in the form of a solution but even when nasally administered in the form of a powder preparation. Thus, an effective non-injection pathway for administering these peptides has yet to be established.

The composition for nasal administration consisting of cyclic peptide and polyvalent metal composition carrier is disclosed in WO 01/52894 A2. It further disclosed that absorption enhancer such as rice flour and starch can be added, and the particle size of those enhancer shall be preferable 250µm or less, more preferable 20 to 180µm. Nevertheless, there was no description regarding the method for the improvement of bioavailability by adding enhancer with about same particle size as that of carrier to the biologically active acidic polypeptide with an isoelectric point of 7 or lower of the present invention.

Accordingly, it is an objective of the present invention to provide a pharmaceutical composition that enables nasal administration of biologically active polypeptides with an isoelectric point in an acidic or neutral pH range. Such polypeptides have a poor bioavailability when administered orally or through other non-inj ection administration routes, have a low solubility in an acidic or neutral pH range, and tend to aggregate even when dissolved in a solution. The pharmaceutical composition is safe and achieves a high bioavailability, while causing no irritancy.

In an effort to find a way to achieve this objective, the present inventors examined possible additives for the potential as a component of the composition for nasal administration of the peptides. First, starch was studied to see if it can help stabilize the peptide as an additive.

Starch, a nutrient abundant in grains, is a material that can be safely used as an additive of the composition of nasal absorption. Starch consists of amylose, which is composed of glucose units joined by α-1,4 linkages to form a straight chain, and amylopectin, which includes α-1,6 linkages and thus is branched.

A polyvalent metal compound is used as a carrier in combination with several different types of starch containing amylose and amylopectin in various proportions as additives to prepare pharmaceutical compositions for nasal absorption. Each pharmaceutical composition was examined for its nasal absorbability. The affect of the particle size of starch as an additive of a pharmaceutical composition on the enhancement of the nasal absorption was also examined.

As a result, the present inventors have found that administration of the following nasal powder composition in a nasal cavity can achieve an improved nasal absorption and can thus provide an effective clinical treatment. Such a composition is prepared by uniformly dispersing and embedding an acidic biologically active polypeptide, such as GLP-1(7-36)NH₂, on the surface of a powdery or crystalline polyvalent metal compound carrier that is either insoluble or little soluble in water and has an average particle size of 100µm or less, such as a compound of divalent or higher metal, for example, calcium compounds, with the aid of an additive such as rice powder (Domyo-ji powder), corn starch, potato starch, and a pregelatinized or partially pregelatinized starch thereof, each containing amylopectin and amylose at a particular proportion.

The present inventors have also found that in the case of using water-insoluble starch as additives and polyvalent metal compound such as calcium carbonate with average particle size of 100µm or less as carrier, water-insoluble starch with particle size smaller than that of carrier shows remarkable absorption facilitation effect in acidic peptide such as GLP-1 (7-36)NH₂. The present invention has been brought to completion based on these findings.

### DISCLOSURE OF THE INVENTION

Accordingly, the present invention provides a pharmaceutical composition for nasal absorption, including a biologically active acidic polypeptide with an isoelectric point of 7 or lower, a carrier that is insoluble or little soluble in water, and an additive for dispersing and embedding the polypeptide on the surface of the carrier.

The present invention also provides a pharmaceutical composition for nasal absorption, including a biologically active polypeptide with its isoelectric point of 7 or lower, that is, in a neutral or an acidic pH range, a polyvalent metal compound carrier, and an additive for dispersing and embedding the polypeptide on the surface of the carrier.

A specific embodiment of the pharmaceutical composition of the present invention for nasal absorption contains, along with the additive with the average particle size of 1µm to 20µm, an effective dosage of the biologically active polypeptide with its isoelectric point within a neutral or an acidic pH range, so that the polypeptide is uniformly dispersed and embedded on the surface of the powder or crystalline polyvalent metal compound carrier having an average particle size of 100µm or less.

Another specific embodiment of the pharmaceutical composition of the present invention for nasal absorption contains peptide incretin, a polyvalent metal compound carrier, and more specifically, the present invention relates to the composition containing carrier that is insoluble or little soluble in water, along with the polyvalent metal compound in fine powder or crystallized form having an average particle size of 100µm or less, and an additive for dispersing and embedding the peptide incretin on the surface of the carrier with the average particle size of 1µm to 20µm.

The mean particle size of an additive presents the mean particle size of water-insoluble or little soluble starch composition on the surface of the carrier, when the formulation is prepared by the method in the present invention using the starch composing with pregelatinized starch or components including pregelatinized starch.

### BRIEF DESCRIPTION OF THE DRAWINGS

Fig. 1 is a diagram showing changes in the plasma concentrations of GLP-1(7-36)NH₂ in Example 2 after subcutaneous administration
Fig. 2 is a diagram showing the time-courses of the plasma concentrations of GLP-1(7-36)NH₂ in Example 2 for nasal administration of additive-free compositions.
Fig. 3 is a diagram showing the time-courses of the plasma concentrations of GLP-1 (7-36)NH₂ in Example 2 for nasal administration of additive-free compositions using sucralfate as carrier.
Fig. 4 is a diagram showing the time-courses of the plasma concentrations of GLP-1 (7-36)NH₂ in Example 2 for nasal administration of additive-contained compositions.

### BEST MODE FOR CARRYING OUT THE INVENTION

As described above, the present invention provides a pharmaceutical composition for nasal absorption that has a high bioavailability and enables nasal administration of biologically active polypeptides having an isoelectric point of 7 or lower that exhibit a low solubility in an acidic or neutral pH range and tend to aggregate even when dissolved in a solution. Such polypeptides have a poor bioavailability and are not suitable for oral administration or administration through other non-injection routes. In one specific, preferred embodiment, the present invention provides a pharmaceutical composition for nasal absorption, containing GLP-1 derivatives such as GLP-1, GLP-1 amide, GLP-1(7-36)NH₂, GLP-1(9-36)NH₂, GLP-1(9-37), GLP-1(7-37), [Val⁸]-GLP-1(7-36)NH₂, [Val⁸]-GLP-1(7-37), [Lys²⁶, ε-NH{γ-Glu(N-α-palmitoyl)}]-GLP-1(7-37), and GLP-2, exendin-3, exendin-4, glucagon, gastric inhibitory peptide (GIP) or insulin.

The high bioavailability of the pharmaceutical composition of the present invention for nasal absorption comes from the biologically active polypeptides dispersed and embedded on the surfaces of a carrier in a stable and uniform manner with the aid of an additive.

When using beta- or partially pregelatinized water-insoluble or water-little soluble starches as additives, using rice starch and corn starch with small average particle size can expanded superficial area and can improved elution, and thus the absorption can be improved. Therefore, the additives with the average particle size of 1µm to 20µm is preferably used to improve the absorption in the present invention.

Thus, the additive for use in the present invention may be any additive that allows the biologically active polypeptide to be dispersed and embedded on the surfaces of the carrier in a stable and uniform manner. For example, starches containing amylopectin and amylose either independently or at a particular proportion can be used as such an additive. Starches obtained from rice, corn or the like are generally classified into "nonglutinous rice"-type starches containing amylopectin and amylose at a ratio of about 7:3 to about 8:2 and "glutinous rice"-type starches composed substantially solely of amylopectin. Specifically, examples of the additive for use in the present invention include rice flour, rice starch, corn starch, potato starch, beta-starch such as rice beta-starch (nonglutinous rice type), rice beta-starch (glutinous rice type), corn beta-starch (nonglutinous rice type), corn beta-starch (glutinous rice type) and potato beta-starch (nonglutinous rice type); pregelatinized rice starch (nonglutinous rice type), pregelatinized rice starch (glutinous rice type), pregelatinized corn starch (nonglutinous rice type), pregelatinized corn starch (glutinous rice type), pregelatinized potato starch (nonglutinous rice type), pregelatinized wheat starch (nonglutinous rice type), and partially pregelatinized starch thereof.

Though little soluble in water, starches can be gelatinized by heating with water to cause its crystal structure to loosen. Both of completely gelatinized starch (pregelatinized starch, or alpha-starch) and partially pregelatinized starch can be used to serve as the additive of the present invention.

Rice flour is made by grinding albumens of rice seeds, which remain after the seeds are stripped of husks and embryos. Rice flour is rich in starch and is commonly used in food and pharmaceutical additives. In the present invention, rice flours without heat treatment that are composed of beta-starch are preferred to heat-treated rice flours containing pregelatinized starch (alpha-starch) or partially pregelatinized starch, however heat-treated rice powder can be used. One example of the preferred rice flour is the Domyo-j i powder containing pregel atinized rice starch. Additionally, not only corn starch that are composed of beta-starch, but also partially pregelatinized or pregelatinized starch (alpha-starch) corn starch can be used in the present invention.

In addition, mixtures of these starches can be used as the additive of the nasal compositions of the present invention.

Also, oligo saccharides, carboxyvinyl polymer, povidone, hydroxypropylcellulose (HPC), xanthan gum, pectin, sodium alginate, powdered gum arabic, and gelatin may also be used as the additive in the present invention.

The biologically active polypeptides used in the composition of the present invention for nasal absorption are those that have an isoelectric point (pI) of 7 or lower. Such polypeptides exhibit a low solubility in an acidic or neutral pH range and tend to aggregate even when dissolved in a solution.

Examples of the preferred biologically active polypeptides are shown below along with their respective isoelectric points:
GLP-1 (pI=5.05); GLP-1 amide (pI=5.47); GLP-1(7-36)NH₂ (pI=6.76); GLP-1 (7-37) (pI=5.53); GLP-1(9-36)NH₂ (pI=4.68); GLP-1(9-37) (pI=4.87); [Val⁸]-GLP-1(7-36)NH₂ (pI=6.76); [Val⁸]-GLP-1(7-37) (pI=5.53); [Lys²⁶, ε-NH{γ-Glu(N-α-palmitoyl)}]-GLP-1(7-37) (pI=4.57); GLP-2 (pI=4.45); exendin-3 (pI=4.96); exendin-4 (pI=4.96); glucagon (pI=6.75); and gastric inhibitory peptide (GIP) (pI=6.92); insulin (pI=5.39).

Other biologically active polypeptides that can be nasally administered may also be used, examples being as follows (in the following, pI signifies an isoelectric point, MW signifies a molecular weight, and all compounds are derived from humans except for exendin-3 and exendin-4) :
calcitonin (pI: 6.72, MW: 3420.88), katacalcin (pI: 5.26, MW: 2436.62), cholecystokinin-12 (pI: 3.93, MW: 1535.71), cholecystokinin-8 (pI: 3.56, MW: 1064.20), corticotropin-lipotropin precursor (pI: 5.22, MW: 8469.32), corticotropin-like intermediate peptide (pI: 3.91, MW: 2309.51), lipotropin-β (pI: 6.17, MW: 9805.94), lipotropin-γ (pI: 4.66, MW: 6074.57), melanotropin-β (pI: 5.57, MW: 2204.40), corticoliberin (pI: 5.09, MW: 4758.49), endothelin-1 (pI: 4.54, MW: 2495.94), endothelin-2 (pI: 4.54, MW: 2550.9), endothelin-3 (pI: 5.38, MW: 2647.09), galanin message-associated peptide (pI: 4.49, MW: 6671.52), gastrin-71 (pI: 5.17, MW: 8066.88), gastrin-34 (pI: 4.25, MW: 3867.26), gastrin-17 (pI: 3.40, MW: 2116.24), gastric inhibitory polypeptide (pI: 6.92, MW: 4983.59), glicentin-related polypeptide (pI: 4.13, MW: 3384.50), glucagon (pI: 6.75, MW: 3482.79), glucagon-like peptide-1 (pI: 5.05, MW: 4167.02), glucagon-like peptide-1 amide (pI:5.47, MW: 4111.50), glucagon-like peptide-1 (7-36) amide (pI: 6.76, MW: 3297.68), glucagon-like peptide-1(7-37) (pI: 5.53, MW: 3355.71), [Val⁸]-glucagon-like peptide-1(7-36) amide (pI:6.76, MW:3326.74), [Val⁸]-glucagon-like peptide-1 (7-37) (pI:5.53, MW:3383.87), [Lys²⁶, ε-NH{γ-Glu(N-α-palmitoyl)}]-GLP-1(7-37) (pI:4.57, MW:3751.2), GLP-1(9-36)NH₂ (pI: 4.68, MW:2933.2), GLP-1(9-37) (pI: 4.87, MW:3090.4), glucagon-like peptide-2 (pI: 4.21, MW: 3922.35), exendin-3 (pI: 4.96, MW: 4202.63), exendin-4 (pI: 4.96, MW:4186.60), insulin β-chain (pI: 6.90, MW: 3429.96), insulin α-chain (pI: 3.79, MW: 2383), insulin (pI: 5.39, MW: 5807.6), progonadoliberin-I (pI: 5.63, MW: 7893.83), gonadoliberin-II (pI: 6.92, MW: 1254.33), gonadoliberin-related peptide-1 (pI: 4.67, MW: 6370.11), neuromedin C (pI: 6.92, MW: 1121.28), insulin-like protein (INSL) A-chain (pI: 6.36, MW: 3542.16), motilin-relatedpeptide E (pI: 4.72, MW: 7433.47), leucine-enkephalin (pI: 5.52, MW: 555.63), methionine-enkephalin (pI: 5.52, MW: 573.66), leumorphin (pI: 6.21, MW: 3351.68), oxytocin (pI: 5.51, MW: 1010.19), neurophysine-1 (pI: 4.94, MW: 9600.88), neurophysine-2 (pI: 5.05, MW: 9787.07), copeptin (pI: 4.11, MW: 4021.46), neuromedin B (pI: 6.74, MW: 1133.29), neuromedin N (pI: 5.52, MW: 617.79), neuropeptide Y (pI: 6.76, MW: 4272.72), neuropeptide AF (pI: 6.05, MW: 1979.18), PACAP-related peptide (pI: 5.38, MW: 4800.32), pancreatic hormone (pI: 6.26, MW: 4182.74), pancreatic icosapeptide (pI: 6.01, MW: 2235.44), peptide YY (pI: 6.77, MW: 4310.80), tyroliberin (pI: 6.74, MW: 380.40), neuroquinine A (pI: 6.74, MW: 1134.32), urocortin (pI: 5.58, MW: 4697.29), urotensin II (pI: 4.37, MW: 1390.59), intestinal peptide (PHM-27) (pI: 6.75, MW: 2986.43), and intestinal peptide-42 (pI: 6.76, MW: 4552.18).

Aside from those shown above, the composition of the present invention may be any biologically active peptide that can be nasally administered.

In the present invention, the carrier for carrying the biologically active polypeptide along with the additive includes carriers insoluble or little soluble in water. For example, polyvalent metal compounds with a valence of 2 or higher selected from aluminum compounds, calcium compounds, magnesium compounds, silicon compounds, iron compounds, or zinc compounds may be used.

Specifically, examples of each type of the polyvalent metal compounds are as follows:

The aluminum compound includes dried aluminum hydroxide gel, chlorohydroxy aluminum, synthetic aluminum silicate, light aluminum oxide, colloidal hydrous aluminum silicate, aluminum magnesium hydroxide, aluminum hydroxide, aluminum hydroxide gel, aluminum sulfate, dihydroxy aluminum acetate, aluminum stearate, natural aluminum silicate, aluminum monostearate, and aluminum potassium sulfate.

The calcium compound includes apatite, hydroxyapatite, calcium carbonate, calcium disodium edetate, calcium chloride, calcium citrate, calcium glycerophosphate, calcium gluconate, calcium silicate, calcium oxide, calcium hydroxide, calcium stearate, calcium tertiary phosphate, calcium lactate, calcium pantothenate, calcium oleate, calcium palmitate, D-calcium pantothenate, calcium alginate, anhydrous calcium phosphate, calcium hydrogen phosphate, calcium dihydrogen phosphate, calcium acetate, calcium saccharate, calcium sulfate, calcium monohydrogen phosphate, calcium para-aminosalicylate, and biologically calcified compounds.

The magnesium compound includes magnesium L-aspartate, magnesium chloride, magnesium gluconate, magnesium aluminosilicate, magnesium silicate, magnesium oxide, magnesium hydroxide, magnesium stearate, magnesium carbonate, magnesium aluminometasilicate, magnesium sulfate, magnesium sodium silicate, and synthetic magnesium sodium silicate.

The silicon compound includes hydrous silicon dioxide, light anhydrous silicic acid, synthetic hydrotalcite, diatomite, and silicon dioxide. The iron compound includes iron sulfate. The zinc compound includes zinc chloride, zinc stearate, zinc oxide, and zinc sulfate.

These polyvalent metal compounds may be used either individually or as a mixture of two or more compounds. Of the polyvalent metal compounds, calcium compounds such as hydroxyapatite, calcium carbonate or calcium lactate produced favorable results.

If the average particle size of the polyvalent metal compound is too large, then the spray performance of the compound worsens and the particles quickly precipitate. Conversely, if the average particle size is too small, then the particles can hardly stay in the nasal cavity and are inhaled into bronchi and lungs. Thus, it is preferred that the polyvalent metal compound has an average particle size of 10 to 100µm, more preferably 20 to 60µm, so that the metal compound can remain efficiently in the nasal cavity.

Although the amount of the biologically active polypeptide in the composition of the present invention to give an effective dose of the polypeptide can vary depending on many factors, including the type of the active substance to be selected, the type of the disease to be treated, desired number of times of administration, age of patients, weight, severity of symptoms, administration route, desired effects, and other factors, it is preferred that, in the case of GLP-1 (7-36) amide for example, the composition of the present invention be nasally administered in a dose that can deliver 50 to 5,000µg of GLP-1(7-36)amide.

Specifically, an effective dose of the biologically active polypeptide is dry-mixed with the carrier (for example, the polyvalent metal compound, including the calcium compound, the aluminum compound, the magnesium compound, the silicon compound, the iron compound, and the zinc compound), and the additive. The carrier, insoluble or little soluble in water, is provided in the form of powder or crystal and has an average particle size of 250µm or less, preferably 100µm or less, and more preferably 20 to 60µm. Alternatively, the components may be wet-mixed with each other in water or in an organic solvent such as ethanol and are then dried. In these manners, the biologically active polypeptide is uniformly dispersed and embedded on the surfaces of the carrier to give the pharmaceutical composition of the present invention for nasal absorption.

The pharmaceutical composition of the present invention for nasal absorption may properly contain carriers commonly used in the formulation of drugs, including lubricant, DPP-IV inhibitor, excipient, thickener, sustainer, stabilizer, anti-oxidant, binder, disintegrator, humectant, coloring agent, fragrance, flavor, suspender, emulsifier, solubilizer, buffering agent, isotonizing agent, surfactant, soothing agent, and various other functional components.

The lubricant includes calcium stearate, magnesium stearate, aluminum stearate, stearic acid and talc.

The stabilizer includes quaternary ammonium salts such as benzalkonium chloride, benzethonium chloride, cetylpyridinium chloride, polyoxyethylene sorbitan fatty acid esters such as polyoxyethylene sorbitan monooleate (Tween 80), and sorbitan fatty acid esters such as sorbitan monooleate (Span 80)

When an acidic biologically active polypeptide, such as GLP-1, that is susceptible to be disintegration by dipeptidylpeptidase IV (DPP-IV) is used, it is preferred that the pharmaceutical composition contain a DPP-IV inhibitor.

Examples of the DPP-IV inhibitor include diprotin A, bacitracin, and isoleucine thiazolidide. While the added amount of the DPP-IV inhibitor may vary depending on the inhibitory activity of each inhibitor, it can be added to the pharmaceutical composition in an amount about 1 to 10,000 times the weight of the biologically active polypeptide, or the active ingredient.

In the production of the composition of the present invention, the amount of the biologically active polypeptide is preferably selected to be in a range of 0.005 to 50%, more preferably in a range of 0.01 to 20%, and still more preferably in a range of 0.1 to 10.0%, assuming the weight of the preparation to be 100%. The amount of the carrier in the composition of the present invention may be any amount suitable for clinical use and is for example in a range of 70 to 99.995%, preferably in a range of 80 to 99.99%, still more preferably in a range of 90 to 99.9%, assuming the weight of the preparation to be 100%. With the amount of the carrier falling within these ranges, a better nasal absorption can be achieved. The amount of the additive in the composition of the present invention is for example in a range of 0. 005 to 50%, preferably in a range of 0.01 to 20%, more preferably in a range of 0.05% to 10.0%, assuming the weight of the preparation to be 100%.

The pharmaceutical composition of the present invention for nasal absorption can be obtained by mixing the polyvalent metal compound carrier, which is insoluble or little soluble in water, the biologically active polypeptide, and the additive. In one example, a powder of GLP-1(7-36)NH₂ to serve as the peptide component is thoroughly mixed with corn starch. The mixture is then placed in a container, to which calcium carbonate is gradually added along with small amounts of purified water to form slurry. The slurry is dried overnight in a desiccator under reduced pressure. The dried products are filtered through a sieve and, if desired, a proper amount of calcium stearate is admixed. This gives the pharmaceutical composition of the present invention.

The pharmaceutical composition of the present invention for nasal absorption can also be obtained by first forming the slurry by corn starch and calcium carbonate added along with small amounts of purified water. The mixture is then placed in a container, to which a powder of GLP-1(7-36)NH₂ is gradually added and kneaded with the water containing benzalkonium chloride. The mixture is dried and filtered and then a proper amount of calcium stearate is admixed. This also gives the pharmaceutical composition of the present invention.

Proper amounts of the resulting pharmaceutical composition for nasal absorption are filled in capsules made of hydroxypropylmethylcellulose (HPMC), starch or gelatin, and the capsules are properly packaged, preferably in a sealed manner. A preferred sealed package is a combination of a blister package with an aluminum package. If necessary, a desiccant may be placed in the aluminum bag. It is desirable that the entire process be carried out at 60 % or below of the humidity.

### EXAMPLES

The present invention will now be described in further detail with reference to examples, which are not intended to limit the scope of the invention in any way.

Unless otherwise specified, testing methods and equipment that are described in the following are used in Examples.

### Main abbreviations used in present specification have following meanings.

| | |
|---|---|
| Fmoc; | fluorenylmethoxycarbonyl |
| Boc; | tert. Butoxycarbonyl |
| Trt; | trytyl |
| Pmc; | pentamethylchromansulfonyl |
| DCC; | dicyclohexylcarbodiimid |
| HOBt; | N-hydroxybezotriazole |
| TFA; | trifluoroacetic acid |
| DIPEA; | diisopropylethylamine |
| DMF; | dimethylformamide |
| NMP; | N-methylpyrrolidone |
| TFE; | trifluoroethanol |

### 1. Peptide analysis by HPLC

The following equipment and conditions are used to perform a reversed-phase HPLC to determine the peptide content in the preparations and for the purpose of peptide analysis in the stability test.

| | |
|---|---|
| Instrumen | SHIMADZU LC-9A system |
| Column | YMC-PROTEIN-RP (4.6mmΦ x 150mm) |
| Column temperature | 40°C |
| Eluant | acetonitrile in 0.1% trifluoroacetic acid, with the concentration of acetonitrile linearly varied in 10 minutes. |
| Flow rate | 1ml/min |
| Detection | UV (214nm) |
| Loaded volume | 50µL |

### 2. Mass-spectrometry

The mass of the peptide was determined by using the following equipment and conditions.

| | |
|---|---|
| Instrument | Finnigan MAT TSQMS |
| Ion source | ESI |
| Ion detection mode | Positive |
| Spray voltage | 4.5kV |
| Capillary temperature | 250°C |
| Mobile phase | 0.2% acetic acid/methanol mixture (1:1) |
| Flow rate | 0.2ml/min |
| Scan range | m/z 550 to 850 |

### 3. Analysis of amino acid sequence

The following instrument was used to analyze amino acid sequences of the peptide:

| | |
|---|---|
| Instrument | Perkin Elmer 477A sequencer |

### 4. Analysis of amino acid composition

The following instrument was used to analyze amino acid composition of the peptide:

| | |
|---|---|
| Instrument | Hitachi L-8500 amino acid analyzer |

### 5. Preservation of samples (stability test)

The samples were stored in the following incubator under the following temperature condition:

| | |
|---|---|
| Instrument | Nagano Science LH-30-14 |
| Temperature settings | 40 ± 2°C, 25 ± 1°C |

### 6. Freeze dry

| | |
|---|---|
| Instrument | LABCONCO Corp., FZ-6 was used. |

### 7. Measurement of concentration in plasma

Following the administration of the pharmaceutical composition, the concentration of GLP-1(7-36)NH₂ in plasma was measured by radioimmunoassay (RIA) or enzymeimmunoassay (ELISA).

### 7-1. Radioimmunoassay (RIA)

A rabbit was sensitized with a composite adjuvant of GLP-1(7-36)NH₂ and bovine thyroglobulin to obtain an antiserum (IgG fraction). The plasma was placed in a test tube along with an anti-GLP-1(7-36)NH₂-rabbit antibody obtained from the antiserum, and the mixture was allowed to stand overnight at 4°C. Subsequently, ¹²⁵I-GLP-1(7-36)NH₂ was added, and the mixture was allowed to stand overnight at 4°C. An anti-rabbit IgG goat serum was then added, and the mixture was allowed to stand for 1 hour at 4°C. The resulting sample was centrifuged and the radioactivity (gamma-ray) of the precipitate was measured by a gamma counter.

### 7-2. Enzymeimmunoassay (ELISA)

Anti-GLP-1(7-36)NH₂ antibody (rabbit polyclonal antibody) was immobilized onto a 96-well plate. The plasma was added to the plate and the reaction was allowed to proceed for 2 hours. After washing the plate, an anti-GLP-1 (7-36)NH₂ antibody (mouse polyclonal antibody) labeled with horseradish peroxidase was added, and the reaction was allowed to proceed for 1 hour at room temperature. After washing the plate, tetramethylbenzidine was added for reaction. The absorbance at 450nm was measured.

### 8. Calculation of isoelectric points (pI) of biologically active polypeptides

ExPASy (Expert Protein Analysis System) Molecular Biology Server, Swiss Institute of Bioinformatics, was used. For the calculation of C-terminal amides, one of Asp or Glu residues in the sequence was replaced with Asn or Gln.

### 9. Synthesis of peptide (incretin peptide)

Using 433A synthesizer (ABI corporation), preserved peptide resin is synthesized according to FastFmoc (0.25mmol) of ABI standard.

### 10. Article size distribution (Measuring particle size of calcium carbonate and starch)

Instrument: Laser Diffraction Analyzer (RODOS SR), SYMPATEC HELOS & RODOS Corp. was used.

### Reference Example 1: Preparation of GLP-1(7-36)NH₂

An expression plasmid pG97ompPR for expressing a fusion protein consisting of an Escherichia coli β-galactosidase derivative (β-gal 97), a 25 amino acid-long linker, and GLP-1(7-37) was prepared (International Patent Publication No. W099/38984). The linker region of the expressed fusion protein includes a cleavage motif for ompT protease (Arg-Arg) and a cleavage motif for Kex2 protease (Pro-Arg) and is cleaved by the proteases at the respective cleavage sites.

In order to obtain the fusion protein, pG97ompPR was introduced in an Escherichia coli strain descended from W3110 strain. The resultant transformants were incubated in a growth medium containing yeast extracts, inorganic salts, and glucose in a 300 L incubator. The cells were incubated until the concentration of the bacteria reached OD660 = 180. The resultant culture solution was processed in a high-pressure homogenizer to destroy cell bodies and then was centrifuged to collect the inclusion body. The precipitate containing the inclusion body was resuspended in deionized water, was centrifuged to wash the inclusion body, and then was resuspended in deionized water to obtain a condensate (about 30L) of the inclusion body with an OD660 of 1000.

To 3. 9L of the inclusion body condensate, 1L of 1M Tris-HCl with an unadjusted pH was added along with 10L of 8M urea. Deionized water was then added to a final volume of 20L. The pH of the resulting solution was adjusted to 6.5 with a 5N hydrochloric acid, and the solution was maintained at 37°C for 2 hours to allow Escherichia coli OmpT protease present in the inclusion body to act on the fusion protein, thereby cleaving off GLP-1(7-37) having 13 amino acids added to its N-terminal (referred to as RHHGP[G], hereinafter). After the reaction was completed, urea powder was added to the reaction mixture to a concentration of 7M and the pH was adjusted to 8.0 with 5N NaOH. The reaction mixture was then filter-pressed to obtain a 30L supernatant. The supernatant was loaded on a SP-Sepharose Big Beads column (140mmID x 160mm, Amersham Pharmacia Biotechnology) equilibrated with a 5M urea/20mM Tris-HCl (pH8.0)/0.1% Tween 80 solution and then was washed with a 0.2M NaCl/20mM Tris-HCl (pH8.0)/0.1% Tween 80 solution. Subsequently, RHHGP[G] was eluted with 0.5M NaCl/20mM Tris-HCl (pH8.0)/0.1% Tween 80 solution and, as a result, a fraction (about 20L) containing about 100g of RHHGP[G] was obtained.

Using purified water (UF water), the resulting RHHGP[G] fraction was adjusted to 5. 0mg/mL. To the solution, 20mM sodium acetate (pH5.2), 5.0µM copper sulfate, 0. 5g/L-ascorbic acid, 1mg/L catalase, 0.1% Tween 80, and 1500unit/mL amidation enzyme were added. Then, the reaction was allowed to proceed at 32°C for 80 minutes while oxygen was blown into the solution to keep the dissolve oxygen concentration at 100%. As a result, the C-terminal of RHHGP[G] was amidated to form an amide form (RHHGP-1). To the solution, Tris-HCl (pH8.0), Tween 80, calcium chloride, and Kex2 protease were added to final concentrations of 20mM, 0.1%, 1mM, and 8,000U/mL, respectively, and the reaction was allowed to proceed at 32°C for two and half an hour.

13 amino acids were removed from the N-terminal of RHHGP-1 to form free GLP-1(7-36)NH₂. An about 10L portion (3.4g/L) of the above solution was diluted (to 26L) with 0.3% Tween 80/20mM Briton Robinson (referred to as BR, hereinafter) buffer (pH4.5) and was loaded onto a cation-exchange chromatography column (90mmID x 400mm, MacroPrep High-S, Biorad) equilibrated with 20mM NaCl/0.3% Tween 80/20mM BR buffer (pH4.5). After washing with the same solution, GLP-1(7-36)NH₂ was eluted with a solution A (20mM BR buffer (pH6.0)/20mM NaCl/0.3% Tween 80) and a solution B (20mM BR buffer (pH7.5)/20mM NaCl/0.3% Tween 80) while the proportion of the solution B in the eluant was linearly varied from 50% to 100% to form a linear gradient.

The resulting fraction with a purity of 98% or higher was diluted with UF water to a GLP-1 (7-36)NH₂ concentration of 6mg/mL and was loaded onto a Prep C18 column (90mmID x 240mm) (Waters) equilibrated with 20mM sodium acetate (pH4.5). After washing with a 10% acetonitrile solution containing 20mM sodium acetate (pH4.5) and 0.2% acetic acid, GLP-1(7-36)NH₂ was. eluted with a 30% acetonitrile solution containing 2% acetic acid to obtain a solution (2.5L) containing 27g GLP-1(7-36)NH₂. Using an evaporator, actetonitrile was removed from the eluate, and the concentration of GLP-1(7-36)NH₂ was adjusted to 10mg/mL with water for injection. The solution was then freeze-dried in RL-903BS freeze-drier (Kyowa Vacuum Engineering Co., Ltd.) to obtain 22g of freeze-dried GLP-1(7-36)NH₂ product.

The obtained product had the following molecular weight and amino acid composition and was thus identified as GLP-1(7-36)NH₂. ESI-MS: 3297.4 (theoretical value: 3297.68). Leu standard Amino acid composition after hydrolysis with 6N hydrochloric acid: Asp 1.0; (1), Thr ; 2.0 (2), Ser;2.7 (3), Glu;4.0 (4), Gly;3.0 (3), Ala ; 4.1 (4), Val ; 2.0 (2), Ile ; 1.0 (1), Leu ; 2.0, Tyr ; 1.0 (1), Phe ; 2.1 (2), Lys; 2.0 (2), His ; 1.0 (1), Arg ; 1.0 (1).

### Reference Example 2: Test for nasal absorption of the solution of GLP-1(7-36)NH₂ pharmaceutical composition using rats

About 10mg of GLP-1(7-36)NH₂ obtained in Reference Example 1, 180mg sucrose, 8mg anhydrous citric acid, and 0.2mg benzalkonium chloride were dissolved in 2mL water to form a sample solution with a concentration of 5mg/mL as measured by reversed-phase HPLC. Male SD rats, aged 7 to 9 weeks and weighing about 250g (Crj: CD, Charles River Japan Inc.,), were kept in a metal cage with a day/night cycle of 12 hours while maintaining the temperature at 22 ± 5°C and the humidity at 30 to 70%. The rats were allowed to feed freely on solid feed and tap water and were fasted for 24 hours prior to the test (3 animals per group).

For nasal administration, a cannula was placed in the femoral artery under anesthesia with pentobarbital, and 5µL of the sample solution was administered in the left nasal cavity with a precision pipette (about 100µg/kg). The blood was collected into a tube containing anticoagulant and enzyme inhibitor at 0, 5, 10, 15, 20, 30, 60, and 90 minutes after administration and was centrifuged to obtain plasma. The concentration of GLP-1 (7-36)NH₂ in the plasma was measured by RIA using anti-GLP-1(7-36)NH₂ antibody. For subcutaneous administration, the sample solution for subcutaneous administration (about 15µg/mL) was subcutaneously administered in the back of the rats using a syringe at a dosage of 1mL/kg. The concentration of GLP-1 (7-36)NH₂ in the plasma was determined in the same manner as in the nasal administration. The results are shown in Table 1 below.

**Table 1 :**

| Nasal absorption of solution of GLP-1 (7-36)NH₂ pharmaceutical composition (in rats) | | | | |
|---|---|---|---|---|
| Admn. Route | Dosage (µg/kg) | Cmax (ng/mL) | AUC (ng·hr/mL) | Bioavailability (%) |
| Nasal | 97±1 | 4.70±3.28 | 2.47±1.44 | 11.2±6.5 |
| Subcutaneous | 15 | 6.68±0.86 | 3.82±0.58 | (100) |
| Mean ± SD (n=3) | | | | |

As can be seen from the results above, the bioavailability was 11.2 ± 6.5% (mean ± SD, n=3), indicating that GLP-1(7-36)NH₂ was effectively absorbed from nasal mucosa.

### Reference Example 3: Stability of the solution of GLP-1(7-36)NH₂ pharmaceutical composition

The sample solution of GLP-1(7-36)NH₂ prepared in Reference Example 2 was stored at 25°C and 40°C.

The results are shown in Table 2 below. As can be seen from the results, formation of small particles was observed after one week in each temperature condition.

**Table 2 :**

| Stability of GLP-1(7-36)NH₂ aqueous solution in 0.4% acetic acid | | | |
|---|---|---|---|
| Time | Evaluation | 25°C | 40°C |
| Initial | Remaining Proportion | (100) | (100) |
| | Appearance | Clear and colorless | Clear and colorless |
| | pH | 2.80 | 2.80 |
| Week 1 | Remaining Proportion | 94.2 | 85.4 |
| | Appearance | Fine particle formed | Fine particle formed |
| | pH | 2.78 | 2.78 |
| Week 2 | Remaining Proportion | 88.5 | 77.3 |
| | Appearance | Fine particles formed | Gelated |
| | pH | 2.78 | 2.71 |
| Peptide concentration: 5mg/mL | | | |
| 0.4% anhydrous citric acid | | | |
| 9% sucrose | | | |
| 0.01% benzalkonium chloride | | | |

Although the results of Reference Example 2 indicate that the solution of the pharmaceutical composition containing GLP-1(7-36)NH₂ has the ability to be nasally absorbed in rats, the physicochemical stability of the solution is low when the solution has a pH of around 2.7. Accordingly, the solution of the pharmaceutical composition is unfavorable when it has such a pH.

### Reference Example 4: Stability of the GLP-1(7-36)NH₂ solution

A 1, 000mL solution A was prepared by adding distilled water to a mixture of 3. 92g phosphoric acid, 2.40g acetic acid, 14.91g potassium chloride, and 2.47g boric acid, and a 1000mL solution B was prepared by adding water to 8.0g of sodium hydroxide. The solution B was added dropwise to the solution A to form 100mL buffer solutions (Britton Robinson (BR) buffer) with respective pH values of pH2.0, pH3.0, pH4.0, pH5.0, pH6.0, pH7.0, and pH9.0.

Meanwhile, about 300mg of GLP-1(7-36)NH₂ prepared in Reference Example 1 was dissolved in 30mL distilled water. The solution (10mg/mL) was divided into 2.0mL aliquots and the BR buffers and 0.1M hydrochloric acid were individually added to the aliquots to form 20mL sample solutions. The samples were then stored in an incubator at 40°C for 1, 4, and 7 days, and the appearance of the samples was observed and the remaining proportions of GLP-1(7-36)NH₂ determined. The results are shown in Table 3 below.

**Table 3 :**

| Stability of GLP-1(7-36)NH₂ aqueous solution at various pH | | | | | | | | | |
|---|---|---|---|---|---|---|---|---|---|
| | | pH | | | | | | | |
| | | 1.2 | 2.0 | 3.0 | 4.0 | 5.0 | 6.0 | 7.0 | 9.0 |
| Initial | Remaining proportion | 100 | 100 | 100 | 100 | 100 | 100 | 100 | 100 |
| | Appearance | C.C. | C.C. | C.C. | C.C. | C.C. | C.C. | C.C. | C.C. |
| Day 1 | Remaining proportion | 61.7 | 4.9 | 33.8 | 99.4 | 98.5 | 27.4 | 65.2 | 95.9 |
| | Appearance | P.P. | P.P. | P.P. | C.C. | C.C. | P.P. | P.P. | C.C. |
| Day 2 | Remaining proportion | 23.1 | 2.5 | 0.9 | 97.4 | 44.0 | 26.0 | 54.1 | 84.3 |
| | Appearance | P.P. | P.P. | P.P. | C.C. | P.P. | P.P. | P.P. | C.C. |
| Day 7 | Remaining proportion | 24.4 | 3.4 | 0.3 | 29.4 | 34.4 | 26.4 | 52.0 | 75.6 |
| | Appearance | P.P. | P.P. | P.P. | P.P. | P.P. | P.P. | P.P. | C.C. |
| C.C.: Clear and colorless | | | | | | | | | |
| P.P.: Partially precipitated | | | | | | | | | |
| Peptide concentration: 1mg/mL | | | | | | | | | |
| Temperature: 40°C | | | | | | | | | |
| pH1.2: 0.1M hydrochloric acid | | | | | | | | | |
| pH2.0∼pH9.0: Briton-Robinson buffer (µ= 0.2) | | | | | | | | | |
| Remaining proportion: Proportion collected in the supernatant | | | | | | | | | |

As shown in Table 3, precipitates were formed after one day in the samples with pH1.2, pH2.0, pH3.0, pH6.0, and pH7.0, after 4 days in the sample with pH 5.0, and after 7 days in the sample with pH4.0. While no precipitates were formed in the sample with pH 9.0, the remaining proportion of GLP-1(7-36)NH₂ decreased to 75.6% after seven days. Thus, it has been shown that GLP-1(7-36)NH₂ exhibits a relatively low stability in any of acidic, neutral, or basic solutions and is therefore not suitable for use in solutions of the pharmaceutical composition.

From such a viewpoint, powder preparations of the composition for nasal administration, an alternative to the solution of the pharmaceutical composition, were tested for the absorbability.

### Example 1: Test for the absorbability of the powder preparation for nasal administration using dogs

Compositions for nasal absorption were prepared by adding different additives as described in the following Preparation Examples 1 through 9 to a mixture of GLP-1 (7-36) NH₂ obtained in Reference Example 1 and a fine powder of calcium carbonate with the average particle size of 50µm to serve as a carrier.

As a control, one additive-free sample of the composition for nasal administration was prepared.

3 beagle dogs, each weighing about 10kg, were used, and the pharmaceutical compositions for nasal absorption as described in Preparation Examples 1 through 9 were nasally administered with a powder nebulizer. The concentration of GLP-1(7-36)NH₂ in the plasma was measured at 0, 5, 10, 20, 30, 45, 60, 90, and 120 minutes after administration. As a control, an additive-free fine powder of GLP-1(7-36)NH₂ using a calcium carbonate carrier was administered. The concentrations of GLP-1(7-36)NH₂ in the plasma were measured by RIA using an anti-GLP-1(7-36)NH₂ antibody. The bioavailability was calculated by comparing the area under the curve (AUC) after nasal administration with the AUC after subcutaneous administration of GLP-1(7-36)NH₂ in saline. The results are shown in Table 4 below.

**Table 4:**

| Evaluation of nasal preparation in dogs | | | | | | |
|---|---|---|---|---|---|---|
| Additives | Dosage (µg/kg) | Cmax (ng/mL) | Tmax (min) | T_{1/2} (hrs) | AUC_{0-∞} (ng·hr/mL) | Bioavailability(%) |
| No additive ¹⁾ | 83.9±3.9 | 2.65±1.72 | 6.7±2.9 | 0.43±0.46 | 1.09±0.22 | 4.3±1.0 |
| 1% Domyo-ji powder | 58.7±2.3 | 2.53±0.73 | 16.7±5.8 | 0.59±0.36 | 2.35±1.66 | 13.4±10.1 |
| 0.1% corn starch | 74.6±7.5 | 3.87±1.22 | 11.7±7.6 | 0.58±0.41 | 2.62±1.01 | 11.4±3.9 |
| 1% corn starch | 58.4±2.8 | 2.78±1.99 | 10.0±0.0 | 0.48±0.30 | 2.47±2.20 | 13.7±11.8 |
| 0.1% nonglutinous rice-type pregelatinized potato starch | 80.1±3.2 | 2.26±0.07 | 13.5±5.8 | 0.59±0.40 | 2.28±1.12 | 9.5±5.0 |
| 1% nonglutinous rice-type pregelatinized potato starch | 52.3±5.7 | 2.64±0.85 | 13.3±0.0 | 0.33±0.19 | 1.40±0.63 | 8.8±4.1 |
| 1% povidone | 52.9±2.4 | 1.96±1.13 | 10.0±0.0 | 0.24±0.07 | 0.91±0.46 | 5.6±2.7 |
| 1% pectin | 53.3±2.8 | 2.57±1.07 | 16.7±11.5 | 0.44±0.28 | 1.05±0.53 | 6.6±3.5 |
| 1% nonglutinous rice-type partially pregelatinized corn starch | 49.7±4.3 | 1.98±1.67 | 16.7±11.5 | 0.44±0.13 | 1.24±0.91 | 8.3±6.2 |
| Subdermal administration | 50 | 20.35±3.48 | 15.0±0.0 | 0.39±0.20 | 5.15±5.13 | 100 |

| | | | | | | |
|---|---|---|---|---|---|---|
| 1) calcium carbonate carrier Mean ± SD (n=3) | | | | | | |

As can be seen from the results above, the bioavailability of the additive-free pharmaceutical composition was about 4%, whereas the administration of the pharmaceutical compositions of the present invention improved the absorption of GLP-1(7-36)NH₂ and resulted in increased bioavailabilities of about 6% to 14%.

### Preparation Example 1: Preparation of pharmaceutical composition containing Domyo-ji powder (1.0%)

As the peptide component, an amount (about 12mg) of the GLP-1(7-36)NH₂ powder equivalent to 10mg of GLP-1(7-36)NH₂ was mixed with 31mg Domyo-ji powder. The powder mixture was placed in a beaker and 2.92g of calcium carbonate (average particle size = 51.9µm) were gradually added. After thoroughly mixing the mixture, purified water was added and the mixture was kneaded. After kneading, the mixture was dried overnight in a desiccator under reduced pressure and the dried products were passed through a 180µm sieve. To the sieved mixture, an amount (29mg) of calcium stearate equivalent to 1.0% of the total weight was admixed to give 2.83g of a powder sample. The GLP-1(7-36)NH₂ content in the powder sample was measured by a reversed-phase HPLC, and an amount (about 226mg) of the powder sample containing 690µg of GLP-1(7-36)NH₂ was filled in #2 capsules to prepare a pharmaceutical composition for nasal absorption.

### Preparation Example 2: Preparation of pharmaceutical composition containing corn starch (0.1%)

As the peptide component, an amount (about 12mg) of the GLP-1(7-36)NH₂ powder equivalent to 10mg of GLP-1(7-36)NH₂ was mixed with 3.5mg of corn starch (*Japanese pharmacopoeia*: average particle size = 13.3µm). The powder mixture was placed in a beaker and 2. 96g of calcium carbonate (average particle size = 51.9µm) were gradually added. After thoroughly mixing the mixture, purified water was added and the mixture was kneaded. After kneading, the mixture was dried overnight in a desiccator under reduced pressure and the dried products were passed through a 180µm sieve. To the sieved mixture, an amount (28mg) of calcium stearate equivalent to 1.0% of the total weight was admixed to give 2.75g of a powder sample. An amount (about 297mg) of the powder sample containing about 1, 000µg of GLP-1 (7-36)NH₂ was filled in #2 capsules to prepare a pharmaceutical composition for nasal absorption.

### Preparation Example 3: Preparation of pharmaceutical composition containing corn starch (1.0%)

As the peptide component, an amount (about 12mg) of the GLP-1(7-36)NH₂ powder equivalent to 10mg of GLP-1(7-36)NH₂ was mixed with 32mg of corn starch (*Japanese pharmacopoeia*: average particle size = 13.3µm). The powder mixture was placed in a beaker and 2.93g of calcium carbonate (average particle size = 51.9µm) were gradually added. After thoroughly mixing the mixture, purified water was added and the mixture was kneaded. After kneading, the mixture was dried overnight in a freeze-dryer under reduced pressure and the dried products were passed through a 180µm sieve. To the sieved mixture, an amount (29mg) of calcium stearate equivalent to 1.0% of the total weight was admixed to give 2. 89g of a powder sample. The GLP-1 (7-36)NH₂ content in the powder sample was measured by a reversed-phase HPLC, and an amount (about 213mg) of the powder sample containing 678µg of GLP-1(7-36)NH₂ was filled in #2 capsules to prepare a pharmaceutical composition for nasal absorption.

### Preparation Example 4: Preparation of pharmaceutical composition containing nonglutinous rice-type pregelatinized potato starch (0.1%)

As the peptide component, an amount (about 12mg) of the GLP-1(7-36)NH₂ powder equivalent to 10mg of GLP-1(7-36)NH₂ was mixed with 3.2mg of nonglutinous rice-type pregelatinized potato starch (AMYCOL HF, NIPPON STARCH CHEMICAL Co., Ltd.). The powder mixture was placed in a beaker and 2.96g of calcium carbonate (average particle size = 51.9µm) were gradually added. After thoroughly mixing the mixture, purified water was added and the mixture was kneaded. After kneading, the mixture was dried overnight in a desiccator under reduced pressure and the dried products were passed through a 180µm sieve. To the sieved mixture, an amount (28mg) of calcium stearate equivalent to 1.0% of the total weight was admixed to give 2.75g of a powder sample. An amount (about 297mg) of the powder sample containing about 1,000µg of GLP-1 (7-36) NH₂ was filled in #2 capsules to prepare a pharmaceutical composition for nasal absorption.

### Preparation Example 5: Preparation of pharmaceutical composition containing nonglutinous rice-type pregelatinized potato starch (1.0%)

As the peptide component, an amount (about 12mg) of the GLP-1(7-36)NH₂ powder equivalent to 10mg of GLP-1 (7-36)NH₂ was mixed with 30mg of nonglutinous rice-type pregelatinized potato starch (AMYCOL HF, NIPPON STARCH CHEMICAL Co., Ltd.). The powder mixture was placed in a beaker and 2.92g of calcium carbonate (average particle size = 51.9µm) were gradually added. After thoroughly mixing the mixture, purified water was added and the mixture was kneaded. After kneading, the mixture was dried overnight in a desiccator under reduced pressure and the dried products were passed through a 180µm sieve. To the sieved mixture, an amount (27mg) of calcium stearate equivalent to 1. 0% of the total weight was admixed to give 2. 77g of a powder sample. The GLP-1(7-36)NH₂ content in the powder sample was measured by a reversed-phase HPLC, and an amount (about 217mg) of the powder sample containing 661µg of GLP-1(7-36)NH₂ was filled in #2 capsules to prepare a pharmaceutical composition for nasal absorption.

### Preparation Example 6: Preparation of pharmaceutical composition containing povidone (1.0%)

As the peptide component, an amount (about 12mg) of the GLP-1(7-36)NH₂ powder equivalent to 10mg of GLP-1(7-36)NH₂ was mixed with 32mg of povidone K30 (The Japanese Standards of Pharmaceutical Additives). The powder mixture was placed in a beaker and 2.93g of calcium carbonate (average particle size = 51.9µm) were gradually added. After thoroughly mixing the mixture, purified water was added and the mixture was kneaded. After kneading, the mixture was dried overnight in a desiccator under reduced pressure and the dried products were passed through a 180µm sieve. To the sieved mixture, an amount (29mg) of calcium stearate equivalent to 1.0% of the total weight was admixed to give 2.84g of a powder sample. The GLP-1(7-36)NH₂ content in the powder sample was measured by a reversed-phase HPLC, and an amount (about 211mg) of the powder sample containing 642µg of GLP-1(7-36)NH₂ was filled in #2 capsules to prepare a pharmaceutical composition for nasal absorption.

### Preparation Example 7: Preparation of pharmaceutical composition containing pectin (1.0%)

As the peptide component, an amount (about 12mg) of the GLP-1(7-36)NH₂ powder equivalent to 10mg of GLP-1 (7-36)NH₂ was mixed with 31mg of pectin (*USP*). The powder mixture was placed in a beaker and 2.93g of calcium carbonate (average particle size = 51.9µm) were gradually added. After thoroughly mixing the mixture, purified water was added and the mixture was kneaded. After kneading, the mixture was dried overnight in a desiccator under reduced pressure and the dried products were passed through a 180µm sieve. To the sieved mixture, an amount (29mg) of calcium stearate equivalent to 1.0% of the total weight was admixed to give 2.88g of a powder sample. The GLP-1(7-36)NH₂ content in the powder sample was measured by a reversed-phase HPLC, and an amount (about 210mg) of the powder sample containing 644µg of GLP-1(7-36)NH₂ was filled in #2 capsules to prepare a pharmaceutical composition for nasal absorption.

### Preparation Example 8: Preparation of pharmaceutical composition containing nonglutinous rice-type pregelatinized corn starch (1.0%)

As the peptide component, an amount (about 12mg) of the GLP-1(7-36)NH₂ powder equivalent to 10mg of GLP-1(7-36)NH₂ was mixed with 31mg of nonglutinous rice-type pregelatinized corn starch (PCS, ASAHI KASEI Co., Ltd.). The powder mixture was placed in a beaker and 2.92g of calcium carbonate (average particle size = 51.9µm) were gradually added. After thoroughly mixing the mixture, purified water was added and the mixture was kneaded. After kneading, the mixture was dried overnight in a desiccator under reduced pressure and the dried products were passed through a 180µm sieve. To the sieved mixture, an amount (28mg) of calcium stearate equivalent to 1.0% of the total weight was admixed to give 2.88g of a powder sample. The GLP-1(7-36)NH₂ content in the powder sample was measured by a reversed-phase HPLC, and an amount (about 211mg) of the powder sample containing 646µg of GLP-1 (7-36)NH₂ was filled in #2 capsules to prepare a pharmaceutical composition for nasal absorption.

### Preparation Example 9: Preparation of additive-free pharmaceutical composition

As the peptide component, an amount (about 36mg) of the GLP-1(7-36)NH₂ powder equivalent to 30mg of GLP-1(7-36)NH₂ was placed in a beaker and 8.88g of calcium carbonate (average particle size = 51.9µm) were gradually added. After thoroughly mixing the mixture, purified water was added and the mixture was kneaded. After kneading, the mixture was dried overnight in a freeze-dryer under reduced pressure and the dried products were passed through a 180µm sieve. To the sieved mixture, an amount (87mg) of calcium stearate equivalent to 1. 0% of the total weight was admixed to give 8.76g of a powder sample. The GLP-1(7-36)NH₂ content in the powder sample was measured by a reversed-phase HPLC, and an amount (about 300mg) of the powder sample containing 937µg of GLP-1 (7-36) NH₂ was filled in #2 capsules to prepare a pharmaceutical composition for nasal absorption.

### Example 2: Test for the absorbability of the powder preparation for nasal administration using cynomolgus monkeys (Test-1)

GLP-1(7-36)NH₂ obtained in Reference Example 1 was used as the biologically active polypeptide and a fine powder of either calcium carbonate or a sucrose sulfate aluminum salt (sucralfate) with an average particle size of 51.9µm was used as the carrier. According to the procedures described in Preparation Examples 10 to 18, different pharmaceutical compositions for nasal absorption were prepared that contain different additives carried by the carrier. The dosage of GLP-1(7-36)NH₂ was adjusted so that about 100µg/animal of GLP-1(7-36)NH₂ was administered at a time. Each additive was added to the pharmaceutical composition in amounts of 0.5%, 1.0%, 10% and 50% with respect to the total weight of the preparation. Two additive-free formulations, one containing calcium carbonate and GLP-1(7-36)NH₂ and the other containing sucralfate and GLP-1 (7-36)NH₂, were prepared to serve as controls.

Using a nasal nebulizer manufactured by UNISIA JECS Co., Ltd., the compositions of Preparation Examples 10 to 18 were individually administered to cynomolgus monkeys, each weighing about 3kg, in their nasal cavities, and the concentrations of GLP-1(7-36)NH₂ in the plasma were measured by RIA and partly by ELISA at 0, 5, 10, 20, 30, 45, 60, 90, and 120 minutes after administration. The bioavailability was determined by comparing the AUC after nasal administration with the AUC after subcutaneous administration.

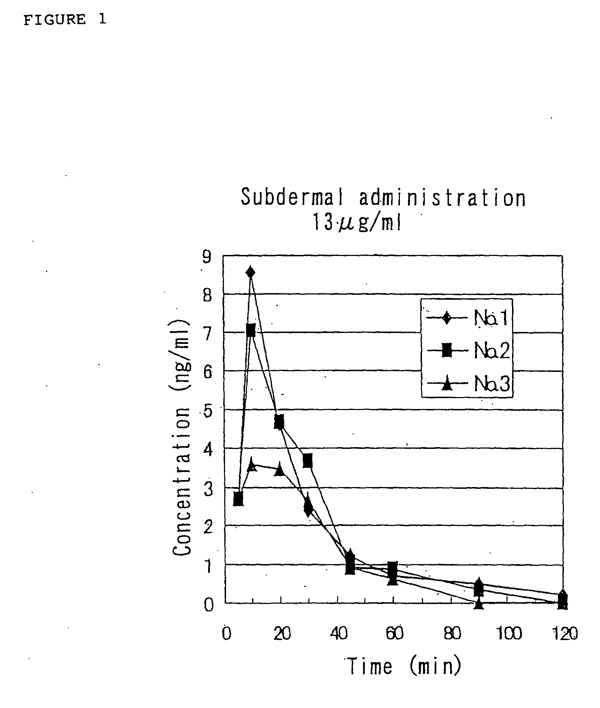

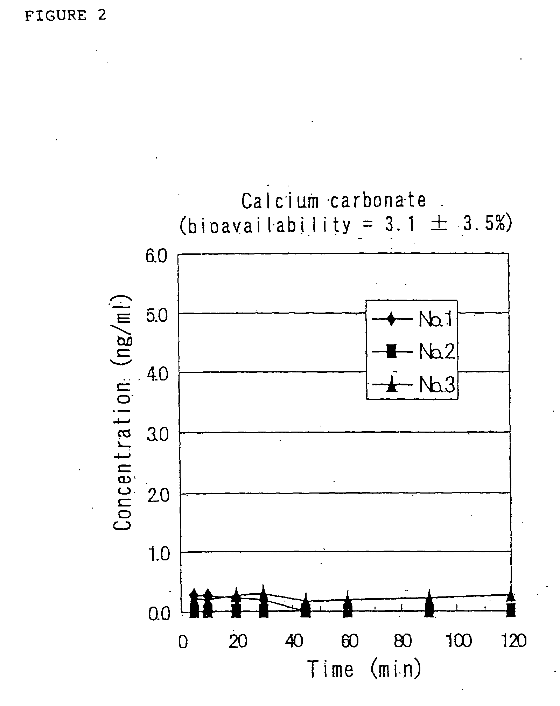

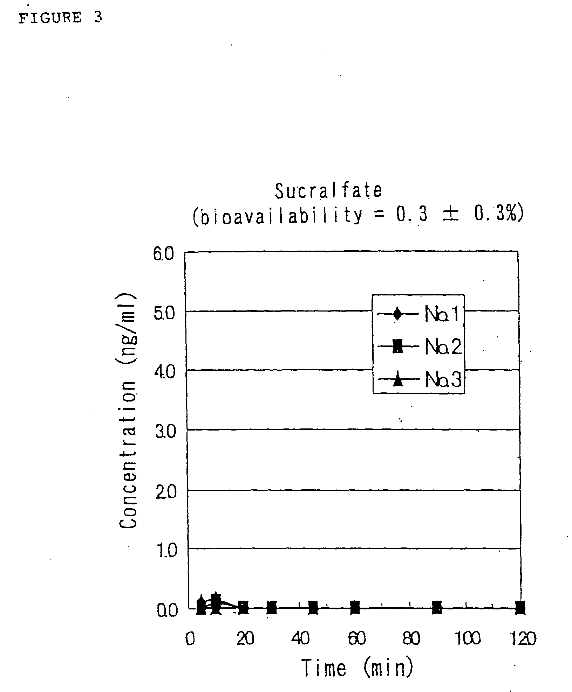

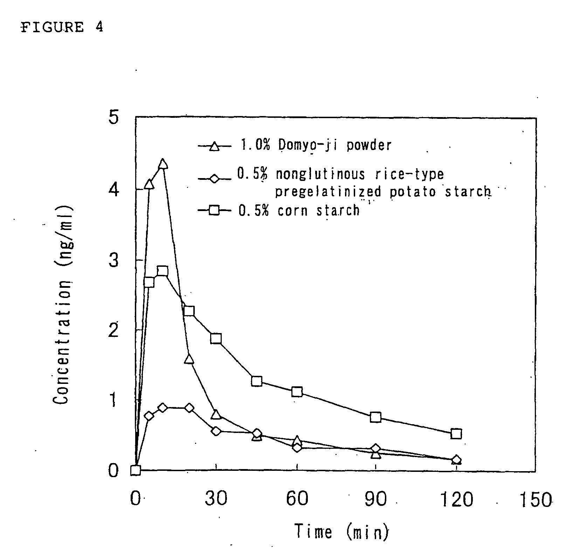

Fig. 1, 2 and 3 show the plasma concentration-time curves of GLP-1 (7-36)NH₂ after subcutaneous administration of GLP-1 (7-36)NH₂ in saline and after intranasal administration of the two additive-free formulations, respectively. Fig. 4 shows the plasma concentration-time curves of GLP-1(7-36)NH₂ after intranasal administration of the respective nasal formulations containing 1% Domyo-ji powder, 0.5% corn starch, or 0.5% nonglutinous rice-type pregelatinized potato starch.

Evaluations of plasma GLP-1(7-36)NH₂ concentrations as determined by RIA and partially by ELISA are shown in Tables 5 and 6, respectively.

**Table 5 :**

| Evaluation of GLP-1 (7-36)NH₂ nasal preparation in cynomolgus monkeys (RIA) | | | | | | |
|---|---|---|---|---|---|---|
| Additives | Dosage (µg/kg) | Cmax (ng/mL) | Tmax (min) | T_{1/2} (hrs) | AUC_{0-∞} (ng·hr/mL) | Bioavailability(%) |
| No additive ¹⁾ | 22±2 | 0.19±0.16 | 20.0±14.1 | -⁴⁾ | 0.19±0.06 | 3.1±3.5 |
| No additive ²⁾ | 24±2 | 0.09±0.08 | 10.0±0.0 | -⁴⁾ | 0.02±0.02 | 0.3±0.3 |
| 1% Domyo-ji powder | 36±2 | 4.51±0.71 | 8.3±2.9 | 0.74±0.53 | 2.05±0.67 | 23.1±7.3 |
| 0.5% corn starch | 37±6 | 3.05±0.93 | 8.3±2.9 | 0.85±0.23 | 3.25±0.95 | 34.7±5.3 |
| 0.5% nonglutinous rice-type pregelatinized potato starch | 35±4 | 0.93±0.40 | 11.7±7.6 | 0.76±0.33 | 1.18±0.92 | 4.1±11.3 |
| 10% HPC³⁾ | 36±5 | 1.71± 0.10 | 10.0±0.0 | 0.74±0.26 | 1.28±0.25 | 14.3±1.3 |
| Subcutaneous administration | 13 | 6.37± 2.54 | 10.0±0.00 | 0.50±0.00 | 3.23±0.5 | (100) |
| Mean ± SD (n=3) | | | | | | |

| | | | | | | |
|---|---|---|---|---|---|---|
| 1) calcium carbonate (Preparation Example 10) | | | | | | |
| 2) sucralfate (Preparation Example 11) | | | | | | |
| 3) kneaded with water (Preparation Example 16) | | | | | | |
| 4) "-" indicates that the plasma concentration was too low to be calculated. | | | | | | |

**Table 6 :**

| Evaluation of GLP-1(7-36)NH₂ nasal preparation in cynomolgus monkey (ELISA) | | | | | |
|---|---|---|---|---|---|
| Additives | Dosage (µg/kg) | Cmax (ng/mL) | Tmax (min) | T_{1/2} (hrs) | AUC_{0-∞} (ng·hr/mL) |
| 10%HPC ⁵⁾ | 35±8 | 0.64±0.29 | 16.7±11.5 | 1.54±0.78 | 0.80±0.22 |
| 10%HPC ³⁾ | 36±5 | 1.11±0.55 | 10.0±0.0 | 0.84±0.10 | 0.85±0.19 |
| 10%HPC ⁶⁾ | 34±2 | 0.49±0.02 | 10.0±0.0 | 0.74±0.19 | 0.41±0.06 |
| 50%HPC ⁷⁾ | 36±4 | 0.27±0.09 | 10.0±0.0 | 1.96±0.24 | 0.53±0.23 |
| 1% Domyo-ji powder | 36±2 | 1.42±0.12 | 5.0±0.0 | 0.61±0.43 | 0.70±0.28 |

| | | | | | |
|---|---|---|---|---|---|
| 3) kneaded with water (Preparation Example 16) | | | | | |
| 5) powder mixture (Preparation Example 15) | | | | | |
| 6) kneaded with ethanol (Preparation Example 17) | | | | | |
| 7) kneaded with water (Preparation Example 18) | | | | | |

As is apparent from Tables 5 and 6 above, each of the two additive-free formulations showed a low nasal absorption of GLP-1(7-36)NH₂ of about 0.3 to 3.1%, whereas each of the pharmaceutical compositions of the present invention significantly facilitated absorption of GLP-1(7-36)NH₂ by the nasal mucosa.

### Preparation Example 10: Preparation of additive-free pharmaceutical composition

As the peptide component, an amount (about 36mg) of the GLP-1(7-36)NH₂ powder equivalent to 30mg of GLP-1(7-36)NH₂ was placed in a beaker and 8.87g of calcium carbonate (average particle size = 51.9µm) were gradually added. After thoroughly mixing the mixture, purified water was added and the mixture was kneaded. After kneading, the mixture was dried overnight in a freeze-dryer under reduced pressure and the dried products were passed through a 180µm sieve. To the sieved mixture, an amount (84mg) of calcium stearate equivalent to 1.0% of the total weight was admixed to give 8.20g of a powder sample. The GLP-1(7-36)NH₂ content in the powder sample was measured by a reversed-phase HPLC, and an amount (about 30mg) of the powder sample containing 100µg of GLP-1 (7-36)NH₂ was filled in #2 capsules to prepare a pharmaceutical composition for nasal absorption.

### Preparation Example 11: Preparation of additive-free pharmaceutical composition

As the peptide component, an amount (about 36mg) of the GLP-1(7-36)NH₂ powder equivalent to 30mg of GLP-1(7-36)NH₂ was placed in a beaker and 8.87g of sucralfate were gradually added. After thoroughly mixing the mixture, purified water was added and the mixture was kneaded. After kneading, the mixture was dried overnight in a freeze-dryer under reduced pressure and the dried products were passed through a 180µm sieve. To the sieved mixture, an amount (84mg) of calcium stearate equivalent to 1.0% of the total weight was admixed to give 8.03g of a powder sample. The GLP-1(7-36)NH₂ content in the powder sample was measured by a reversed-phase HPLC, and an amount (about 30mg) of the powder sample containing 100µg of GLP-1(7-36)NH₂ was filled in #2 capsules to prepare a pharmaceutical composition for nasal absorption.

### Preparation Example 12: Preparation of pharmaceutical composition containing Domyo-ji powder (1.0%)

As the peptide component, an amount (about 36mg) of the GLP-1(7-36)NH₂ powder equivalent to 30mg of GLP-1(7-36)NH₂ was mixed with 89mg of Domyo-ji powder. The powder mixture was placed in a beaker and 8.78g of calcium carbonate were gradually added. After thoroughly mixing the mixture, purified water was added and the mixture was kneaded. After kneading, the mixture was dried overnight in a freeze-dryer under reduced pressure and the dried products were passed through a 180µm sieve. To the filtered mixture, an amount (84mg) of calcium stearate equivalent to 1.0% of the total weight was admixed to give 8.11g of a powder sample. The GLP-1(7-36)NH₂ content in the powder sample was measured by a reversed-phase HPLC, and an amount (about 30mg) of the powder sample containing 100µg of GLP-1(7-36)NH₂ was filled in #2 capsules to prepare a pharmaceutical composition for nasal absorption.

### Preparation Example 13: Preparation of pharmaceutical composition containing corn starch (0.5%)

As the peptide component, an amount (about 36mg) of the GLP-1(7-36)NH₂ powder equivalent to 30mg of GLP-1(7-36)NH₂ was mixed with 45mg of corn starch (*Japanese Pharmacopoeia*: average particle size = 13.3µm). The powder mixture was placed in a beaker and 8.83g of calcium carbonate (average particle size = 50µm) were gradually added. After thoroughly mixing the mixture, purified water was added and the mixture was kneaded. After kneading, the mixture was dried overnight in a freeze-dryer under reduced pressure and the dried products were passed through a 180µm sieve. To the sieved mixture, an amount (88mg) of calcium stearate equivalent to 1.0% of the total weight was admixed to give 8.46g of a powder sample. The GLP-1 (7-36)NH₂ content in the powder sample was measured by a reversed-phase HPLC, and an amount (about 30mg) of the powder sample containing 98µg of GLP-1(7-36)NH₂ was filled in #2 capsules to prepare a pharmaceutical composition for nasal absorption.

### Preparation Example 14: Preparation of pharmaceutical composition containing nonglutinous rice-type pregelatinized potato starch (0.5%)

As the peptide component, an amount (about 36mg) of the GLP-1(7-36)NH₂ powder equivalent to 30mg of GLP-1(7-36)NH₂ was mixed with 45mg of nonglutinous rice-type pregelatinized potato starch (AMYCOL HF, NIPPON STARCH CHEMICAL Co., Ltd.). The powder mixture was placed in a beaker and 8.83g of calcium carbonate (average particle size = 51.9µm) were gradually added. After thoroughly mixing the mixture, purified water was added and the mixture was kneaded. After kneading, the mixture was dried overnight in a freeze-dryer under reduced pressure and the dried products were passed through a 180µm sieve. To the sieved mixture, an amount (89mg) of calcium stearate equivalent to 1.0% of the total weight was admixed to give 8.47g of a powder sample. The GLP-1(7-36)NH₂ content in the powder sample was measured by a reversed-phase HPLC, and an amount (about 30mg) of the powder sample containing 101µg of GLP-1(7-36)NH₂ was filled in #2 capsules to prepare a pharmaceutical composition for nasal absorption.

### Preparation Example 15: Preparation of pharmaceutical composition (powder mixture) containing hydroxyl-propylcellulose (HPC, 10%)

As the peptide component, an amount (about 12mg) of the GLP-1(7-36)NH₂ powder equivalent to 10mg of GLP-1(7-36)NH₂ was triturated with 300mg of HPC (*Japanese Pharmacopoeia*) to obtain a powder mixture. The powder mixture was similarly triturated with 2.69g of calcium carbonate (average particle size = 51.9pm) until uniform. The mixture was then passed through a 180µm sieve. To the sieved mixture, an amount (28mg) of calcium stearate equivalent to 1.0% of the total weight was admixed to give 2.76g of a powder sample. The GLP-1(7-36)NH₂ content in the powder sample was measured by a reversed-phase HPLC, and an amount (about 30mg) of the powder sample containing 100µg of GLP-1 (7-36)NH₂ was filled in #2 capsules to prepare a pharmaceutical composition for nasal absorption.

### Preparation Example 16: Preparation of pharmaceutical composition (mixture kneaded with water) containing HPC (10%)

As the peptide component, an amount (about 12mg) of the GLP-1(7-36)NH₂ powder equivalent to 10mg of GLP-1(7-36)NH₂ was triturated with 300mg of HPC (*Japanese Pharmacopoeia*) to obtain a powder mixture. The powder mixture was similarly triturated with 2.69g of calcium carbonate (average particle size = 51.9µm) until uniform. Purified water was then added to the mixture to form the mixture into a paste. The paste was thoroughly mixed and was dried overnight in a desiccator under reduced pressure. The dried mixture was passed through a 180µm sieve. To the sieved mixture, an amount (28mg) of calcium stearate equivalent to 1.0% of the total weight was admixed to give 2.70g of a powder sample. The GLP-1(7-36)NH₂ content in the powder sample was measured, and about 30mg of the powder sample containing 100µg of GLP-1 (7-36)NH₂ was filled in #2 capsules to prepare a pharmaceutical composition for nasal absorption.

### Preparation Example 17: Preparation of pharmaceutical composition (mixture kneaded with ethanol) containing HPC (10%)

As the peptide component, an amount (about 12mg) of the GLP-1(7-36)NH₂ powder equivalent to 10mg of GLP-1(7-36)NH₂ was triturated with 300mg of HPC (Japanese Pharmacopoeia) to obtain a powder mixture. The powder mixture was similarly triturated with 2.69g of calcium carbonate (average particle size = 51.9µm) until uniform. Ethanol was then added to the mixture to form the mixture into a paste. The paste was kneaded and was dried overnight in a desiccator under reduced pressure. The dried mixture was passed through a 180µm sieve. To the sieved mixture, an amount (28mg) of calcium stearate equivalent to 1.0% of the total weight was admixed to give 2.73g of a powder sample. The GLP-1(7-36)NH₂ content in the powder sample was measured, and about 30mg of the powder sample containing 100µg of GLP-1 (7-36) NH₂ was filled in #2 capsules to prepare a pharmaceutical composition for nasal absorption.

### Preparation Example 18: Preparation of pharmaceutical composition containing HPC (50%)

As the peptide component, an amount (about 12mg) of the GLP-1(7-36)NH₂ powder equivalent to 10mg of GLP-1(7-36)NH₂ was triturated with 1. 5g of HPC (Japanese Pharmacopoeia) to obtain a powder mixture. The powder mixture was similarly triturated with 1.49g of calcium carbonate (average particle size = 51.9µm) until uniform. Purified water was then added to the mixture to form the mixture into a paste. The paste was kneaded and was dried overnight in a desiccator under reduced pressure. The resulting powder was passed through a 180µm sieve. To the sieved mixture, an amount (27mg) of calcium stearate equivalent to 1.0% of the total weight was admixed to give 2.54g of a powder sample. The GLP-1(7-36)NH₂ content in the powder sample was measured, and about 30mg of the powder sample containing 100µg of GLP-1(7-36)NH₂ was filled in #2 capsules to prepare a pharmaceutical composition for nasal absorption.

### Example 3: Absorbability test of the powder preparation for nasal administration using cynomolgus monkeys (Test-2)

In this example, we have examined the nasal absorbability of GLP-1 (7-36)NH₂ by administering compositions containing the partially pregelatinized starch, hydroxypropylcellulose-SSL (HPC-SSL), the mixture·of the partially pregelatinized starch/HPC-SSL or cornstarch (amount of 1.0 to 10%) as an additive.

Using a nasal nebulizer manufactured by UNISIA JECS Co., Ltd., the composition of Preparation Example 19 to 27 were individually administered to cynomolgus monkeys, each weighing about 2 to 4kg, in their nasal cavities, and the concentration of GLP-1(7-36)NH₂ in the plasma were measured by RIA at 0, 5, 10, 15, 30, 60, 90 and 120 minutes after administration. The bioavailability was determined by comparing the AUC after nasal administration with the AUC after subcutaneous administration.

The results were shown in Table 7 below. As is apparent from Table 7, the preparations containing corn starch in the wide range as 1.0 to 10% showed the effective nasal absorption facilitation effect of GLP-1(7-36)NH₂ in comparison with the additive-free formulation. Further, the formulation containing the partially pregelatinized corn starch, which remains partially water-insoluble, also showed the effective nasal absorption of GLP-1(7-36)NH₂. By using hardly water-soluble HPC-SSL as the additive in the pharmaceutical compositions, a certain absorption facilitation effect of GLP-1(7-36)NH₂ was observed. Though the absorption enhancement effect in combination use of HPC-SSL and partially pregelatinized starch was not observed, the nasal absorption of GLP-1(7-36)NH₂ was enhanced in comparison with the additive-free formulation.

**Table 7:**

| Evaluation of GLP-1 (7-36)NH₂ nasal preparation in cynomolgus monkeys (RIA) | | | | | | | |
|---|---|---|---|---|---|---|---|
| Additives (%) | | Dosage (µg/kg) | Cmax (ng/mL) | Tmax (min) | T_{1/2} (hrs) | AUC_{0-∞} (ng·hr/mL) | Bioavailability (%) |
| No additive | - | 88.9±20.0 | 1.47±0.70 | 10.3±0.6 | 0.45±0.21 | 50.80±21.31 | 4.0±1.9 |
| Corn starch | 1% | 100.9±23.2 | 2.53±2.33 | 11.0±1.7 | 0.37±0.08 | 68.90±53.28 | 4.5±2.9 |
| | 5% | 103.5 | 6.87 | 10.0 | 0.65 | 258.37 | 16.7 |
| | 10% | 111.1±19.9 | 3.21±1.13 | 10.0±0.0 | 0.49±0.26 | 127.77±41.02 | 7.6±1.2 |
| HPC (SSL) | 0.1% | 101.9±19.6 | 3.20±1.89 | 10.3±0.6 | 0.34±0.06 | 104.52±56.23 | 6.6±2.5 |
| | 0.5% | 93.7±25.6 | 2.01±0.44 | 10.0±5.0 | 0.57±0.43 | 65.47±15.24 | 4.9±1.3 |
| | 1% | 99.1±25.1 | 1.51±0.96 | 19.0±10.8 | 0.43±0.12 | 62.62±37.66 | 5.0±4.4 |
| Partially pregelatinized corn starch | 1% | 102.2±8.6 | 5.57±1.47 | 12.3±2.5 | 0.36±0.28 | 189.82±83.09 | 12.4±5.0 |
| Pregelatinized corn starch/HPC-SSL | 5%/0.1% | 112.6±31.1 | 4.98±2.23 | 10.0±0.0 | 0.39±0.10 | 155.71±58.06 | 9.1±1.1 |

### Preparation Example 19: Preparation of additive-free pharmaceutical composition containing benzalkonium chloride (0.01%)

As the peptide component, an amount (about 36mg) of the GLP-1(7-36)NH₂ powder equivalent to 30.6mg of GLP-1(7-36)NH₂ was gradually mixed with 2.93g of calcium carbonate (mean particle size: 53.6µm). After thoroughly mixing the mixture, the solution containing 0.3mg of benzalkonium chloride was added and then purified water was further added, and the resultant mixture was kneaded. After kneading, the mixture was dried overnight in a freeze-dryer under reduced pressure and the dried products were passed through 180µm sieve. To the sieved mixture, an amount (about 30mg) of calcium stearate equivalent to 1.0% of the total weight was admixed to give 2.95g of a powder sample. The GLP-1(7-36)NH₂ content in the powder sample was measured by a reversed-phase HPLC, and 30mg of the powder sample containing 311µg of GLP-1(7-36)NH₂ was prepared as a pharmaceutical composition for nasal absorption.

### Preparation Example 20: Preparation of pharmaceutical composition containing corn starch (1.0%) and benzalkonium chloride (0.01%)

About 2. 91g of calcium carbonate (average particle size: 53.6µm) was mixed with about 29mg of corn starch (average particle size: 13.3µm). After thoroughly mixing the mixture, purified water was added and the mixture was kneaded. After kneading, the mixture was dried overnight in a freeze-dryer under reduced pressure and passed through 180µm sieve to obtain the dried products. As the peptide component, an amount (about 36mg) of the GLP-1(7-36)NH₂ powder equivalent to 29.9mg of GLP-1 (7-36) NH₂ was mixed with the dried products obtained above. After thoroughly mixing the mixture, the solution containing 0.3mg of benzalkonium chloride was added and then purified water was further added and the resultant mixture was kneaded. After kneading, the mixture was dried overnight in a freeze-dryer under reduced pressure and the dried products were passed through 180µm sieve. To the sieved mixture, an amount (about 29mg) of calcium stearate equivalent to 1.0% of the total weight was admixed to give about 2. 88g of a powder sample. The GLP-1(7-36)NH₂ content in the powder sample was measured by a reversed-phase HPLC, and 30mg of the powder sample containing 312µg of GLP-1(7-36)NH₂ was prepared as a pharmaceutical composition for nasal absorption.

### Preparation Example 21: Preparation of pharmaceutical composition containing corn starch (5.0%) and benzalkonium chloride (0.01%)

About 2.78g of calcium carbonate (average particle size: 53. 6µm) was mixed with about 150mg of cornstarch (average particle size: 13.3µm). After thoroughly mixing the mixture, purified water was added and the mixture was kneaded. After kneading, the mixture was dried overnight in a freeze-dryer under reduced pressure and passed through 180µm sieve to obtain the dried products. As the peptide component, an amount (about 35mg) of the GLP-1(7-36)NH₂ powder equivalent to 29.6mg of GLP-1(7-36)NH₂ was mixed with the dried products obtained above. After thoroughly mixing the mixture, the solution containing 0.3mg of benzalkonium chloride was added and then purified water was further added and the resultant mixture was kneaded. After kneading, the mixture was dried overnight in a freeze-dryer under reduced pressure and the dried products were passed through 180µm sieve. To the sieved mixture, an amount (about 30mg) of calcium stearate equivalent to 1.0% of the total weight was admixed to give about 2.89g of a powder sample. The GLP-1 (7-36)NH₂ content in the powder sample was measured by a reversed-phase HPLC, and 30mg of the powder sample containing 307µg of GLP-1(7-36)NH₂ was prepared as a pharmaceutical composition for nasal absorption.

### Preparation Example 22: Preparation of pharmaceutical composition containing corn starch (10.0%) and benzalkonium chloride (0.01%)

About 2. 63g of calcium carbonate (average particle size: 53.6µm) was mixed with about 301mg of corn starch (average particle size: 13.3µm). After thoroughly mixing the mixture, purified water was added and the mixture was kneaded. After kneading, the mixture was dried overnight in a freeze-dryer under reduced pressure and passed through 180µm sieve to obtain the dried products. As the peptide component, an amount (about 36mg) of the GLP-1(7-36)NH₂ powder equivalent to 30.6mg of GLP-1(7-36)NH₂ was mixed with the dried products obtained above. After thoroughly mixing the mixture, a solution containing 0.3mg of benzalkonium chloride was added and then purified water was further added and the resultant mixture was kneaded. After kneading, the mixture was dried overnight in a freeze-dryer under reduced pressure and the dried products were passed through meshed sieve of 180µm. To the sieved mixture, an amount (about 27mg) of calcium stearate equivalent to 1.0% of the total weight was admixed to give about 2.69g of a powder sample. The GLP-1(7-36)NH₂ content in the powder sample was measured by a reversed-phase HPLC, and 30mg of the powder sample containing 341µg of GLP-1(7-36)NH₂ was prepared as a pharmaceutical composition for nasal absorption.

### Preparation Example 23: Preparation of pharmaceutical composition containing HPC-SSL (0.1%) and benzalkonium chloride (0.01%)

To about 2.93g of calcium carbonate (average particle size: 53.6µm) was added a solution containing about 3mg of HPC-SSL and then purified water was further added and the mixture was kneaded. After kneading, the mixture was dried overnight in a freeze-dryer under reduced pressure and passed through 180µm sieve to obtain the dried products. As the peptide component, an amount (about 36mg) of the GLP-1 (7-36)NH₂ powder equivalent to 30. 4mg of GLP-1 (7-36)NH₂ was mixed with the dried products obtained above. After thoroughly mixing the mixture, a solution containing 0.3mg of benzalkonium chloride was added and then purified water was further added and the resultant mixture was kneaded. After kneading, the mixture was dried overnight in a freeze-dryer under reduced pressure and the dried products were passed through 180µm sieve. To the sieved mixture, an amount (about 30mg) of calcium stearate equivalent to 1.0% of the total weight was admixed to give about 2.94g of a powder sample. The GLP-1(7-36)NH₂ content in the powder sample was measured by a reversed-phase HPLC, and 30mg of the powder sample containing 310µg of GLP-1(7-36)NH₂ was prepared as a pharmaceutical composition for nasal absorption.

### Preparation Example 24: Preparation of pharmaceutical composition containing HPC-SSL (0.5%) and benzalkonium chloride (0.01%)

To about 2.92g of calcium carbonate (average particle size: 53.6µm) was added a solution containing about 15mg of HPC-SSL and then purified water was further added and the mixture was kneaded. After kneading, the mixture was dried overnight in a freeze-dryer under reduced pressure and passed through 180µm sieve to obtain the dried products. As the peptide component, an amount (about 36mg) of the GLP-1(7-36)NH₂ powder equivalent to 30.5mg of GLP-1 (7-36)NH₂ was mixed with the dried products obtained above. After thoroughly mixing the mixture, a solution containing 0.3mg of benzalkonium chloride was added and then purified water was further added and the resultant mixture was kneaded. After kneading, the mixture was dried overnight in a freeze-dryer under reduced pressure and the dried products were passed through 180µm sieve. To the sieved mixture, an amount (about 30mg) of calcium stearate equivalent to 1.0% of the total weight was admixed to give about 2.93g of a powder sample. The GLP-1(7-36)NH₂ content in the powder sample was measured by a reversed-phase HPLC, and 30mg of the powder sample containing 312µg of GLP-1(7-36)NH₂ was prepared as a pharmaceutical composition for nasal absorption.

### Preparation Example 25: Preparation of pharmaceutical composition containing HPC-SSL (1.0%) and benzalkonium chloride (0.01%)

To about 2.90g of calcium carbonate (average particle size: 53.6µm) was added a solution contains about 30mg of HPC-SSL and then purified water was further added and the mixture was kneaded. After kneading, the mixture was dried overnight in a freeze-dryer under reduced pressure and passed through 180µm sieve to obtain the dried products. As the peptide component, an amount (about 36mg) of the GLP-1 (7-36)NH₂ powder equivalent to 30. 6mg of GLP-1(7-36)NH₂ was mixed with the dried products obtained above. After thoroughly mixing the mixture, a solution containing 0.3mg of benzalkonium chloride was added and then purified water was further added and the resultant mixture was kneaded. After kneading, the mixture was dried overnight in a freeze-dryer under reduced pressure and the dried products were passed through 180µm sieve. To the sieved mixture, an amount (about 28mg) of calcium stearate equivalent to 1.0% of the total weight was admixed to give about 2.85g of a powder sample. The GLP-1(7-36)NH₂ content in the powder sample was measured by a reversed-phase HPLC, and 30mg of the powder sample containing 322µg of GLP-1 (7-36)NH₂ was prepared as a pharmaceutical composition for nasal absorption.

### Preparation Example 26: Preparation of pharmaceutical composition containing partially pregelatinized corn starch (1.0%) and benzalkonium chloride (0.01%)

To about 2.90g of calcium carbonate (average particle size: 53.6µm) was added a solution containing about 30mg of partially pregelatinized corn starch and then purified water was further added and the mixture was kneaded. After kneading, the mixture was dried overnight in a freeze-dryer under reduced pressure and passed through 180µm sieve to obtain the dried products. As the peptide component, an amount (about 37mg) of the GLP-1(7-36)NH₂ powder equivalent to 30.8mg of GLP-1(7-36)NH₂ was mixed with the dried products obtained above. After thoroughly mixing the mixture, a solution containing 0.3mg of benzalkonium chloride was added and then purified water was further added and the resultant mixture was kneaded. After kneading, the mixture was dried overnight in a freeze-dryer under reduced pressure and the dried products were passed through 180µm sieve. To the sieved mixture, an amount (about 29mg) of calcium stearate equivalent to 1.0% of the total weight was admixed to give about 2.90g of a powder sample. The GLP-1(7-36)NH₂ content in the powder sample was measured by a reversed-phase HPLC, and 30mg of the powder sample containing 319µg of GLP-1(7-36)NH₂ was prepared as a pharmaceutical composition for nasal absorption.

### Preparation Example 27: Preparation of pharmaceutical composition containing corn starch (5.0%), HPC-SSL (0.1%) and benzalkonium chloride (0.01%)

About 2. 78g of calcium carbonate (average particle size: 53.6µm) was mixed with about 151mg of corn starch (average particle size: 13.3µm) and the mixture was thoroughly mixed. Then, a solution containing about 3mg of HPC-SSL was added and then purified water was further added to the mixture and the resultant mixture was kneaded. After kneading, the mixture was dried overnight in a freeze-dryer under reduced pressure and passed through 180µm sieve to obtain the dried products. As the peptide component, an amount (about 36mg) of the GLP-1 (7-36)NH₂ powder equivalent to 30.3mg of GLP-1 (7-36)NH₂ was mixed with the dried products obtained above. After thoroughly mixing the mixture, a solution containing 0.3mg of benzalkonium chloride was added and then purified water was further added and the resultant mixture was kneaded. After kneading, the mixture was dried overnight in a freeze-dryer under reduced pressure and the dried products were passed through 180µm sieve. To the sieved mixture, an amount (about 30mg) of calcium stearate equivalent to 1.0% of the total weight was admixed to give about 2.93g of a powder sample. The GLP-1(7-36)NH₂ content in the powder sample was measured by a reversed-phase HPLC, and 30mg of the powder sample containing 310µg of GLP-1(7-36)NH₂ was prepared as a pharmaceutical composition for nasal absorption.

### Example 4: The effect to the nasal absorption by starch addition of incretin polypeptides

This example examined whether nasal absorption of the acidic incretin hormones other than GLP-1(7-36)NH₂ would be enhanced by addition of starch, using decreasing effect in the blood glucose level in rat as an index.

Male CD (SD) rats weighing about 300g were anaesthetized with pentobarbital. The front edge of tip for pipette (GPS-250, RAININ) was filled with 3mg of a pharmaceutical composition (containing about 100µg of polypeptide) prepared by the Preparation Example described later, and the tip was charged in the syringe, and then the composition was sprayed in the nasal cavity of the rats together with 1ml of air. Then, 0.5g/kg of glucose was intravenously administered in the tail of the rats 5 minutes after nasal administration of the composition. The blood samples were collected from the aorta 15 minutes after nasal administration of the composition (10 minutes after glucose administration). The blood glucose level was detected using the measurement instrument for blood glucose level (FREESTYLE-KISSEI; Kissei Pharmaceutical Co., Ltd.)

The results are shown in Table 8 below. As can be seen from the results, the blood glucose level of the control group without administration of incretin hormones was 196mg/dl (average value of 3 rats). As for the composition containing incretin hormones, the blood glucose level fell as compared with the control group. Moreover, also the incretin hormones, the blood glucose level fell when composition containing 5% corn starch is added compared with when 5% corn starch is not added.

These results clearly show that it is possible to make much incretin hormones to be absorbed with the pharmaceutical composition of the present invention, and the absorbability of the incretin hormones was promoted by using starch as additive for the composition.

**Table 8:**

| The blood glucose level decreasing effect of by administering incretin hormones | | | |
|---|---|---|---|
| Polypeptide | Composition | Mean | S.D. |
| Control | - | 196 | 12 |
| GLP-1(7-37) | No additive | 170 | 4 |
| | 5% corn starch | 146 | 12 |
| Exendin-4 | No additive | 166 | 6 |
| | 5% corn starch | 151 | 12 |
| [Val⁸]-GLP-1(7-37) | No additive | 168 | 12 |
| | 5% corn starch | 147 | 10 |
| [Lys²⁶, ε-NH{γ-Glu(N-α-palmitoyl)}]-GLP-1(7-37) | No additive | 184 | 8 |
| | 5% corn starch | 154 | 12 |

### Reference Example 5: Synthesis of [Lys-26, ε-NH(γ-Glu(N-α-palmitoyl)}]-GLP-1(7-37)

To 5mg (6.9mmol) of swelling Cl-Trt(2-Cl) resin with methylene chloride (30ml) was added a methylene chloride solution of Fmoc-Glu-OtBu (5.8g, 13.7mmol) and DIPEA (1.5g, 11.8mmol), and the mixture was stirred gently for 30 minutes. The resin was collected by filtration and washed with methylene chloride, isopropanol and methylene chloride, respectively: Then, a mixed solution of 20% piperidine/DMF (30ml) was added to the resultant resin, and the mixture was stirred gently for 20 minutes. The resin was collected by filtration and washed with DMF, isopropanol and methylene chloride, respectively, and dried. The obtained H-Glu(α-OtBu)-Trt(2-Cl) resin (6.1g, 6.9mmol) was suspended in a mixture solution of N-methyl pyrrolidone (NMP) /methylene chloride (1:1, 30ml) and to this mixture were added 3 equivalent of palmitic acid (5.3g, 20.7mmol), HOBt (2.8g, 20.7mmol) and warter soluble dicyclohexylcarbodiimide (DCC: 4.0g, 20.7mmol), and then, the mixture was stirred gently for over night. The resultant resin was washed with methylene chloride, NMP and methylene chloride respectively and dried. To the obtained palmitoyl-Glu(α-OtBu)-Trt(2-Cl) resin (8g, 6.9mmol) was added a mixture solution of acetic acid/TFE/methylene chloride (1:2:7, 20ml) and the mixture was stirred gently for 20 minutes. The resin was filtrated and washed with 10ml of TFE, and the filtrate was concentrated. The resultant residue was treated with.hexane to obtain precipitate. The obtained precipitate was recrystallized from methylene chloride/hexane to give 2.0g (yield: 66%) of glutamic acid derivative to introduce for the side chain of Lys at 26 position, that is, palmitoyl-Glu(α-OtBu). ESI-MS: [M+H] 442.3; M+Na] 462.4 (theoretical value: 441.3 [M]).

Then, Fmoc-His(Trt)-Ala-Glu(OtBu)-Gly-Thr(Trt)-Phe-Thr(Trt)-Ser(tBu)-Asp(tBu)-Val-Ser(tBu)-Ser(tBu)-Tyr(tBu)-Leu-Glu(OtBu)-Gly-Gln(Trt)-Ala-Ala-Lys(4-methyltrityl)-Glu(OtBu)-Phe-Ile-Ala-Trp-Leu-Val-Arg(Pmc)-Gly-Arg(Pmc)-Gly-Wang resin was constructed by Fmoc method (0.25mmol protocol) using the automatic peptide synthesizer 433A (ABI Co., Ltd.). The obtained resin was treated with a mixture solution of 1% TFA/5% TIPS/methylene chloride (30ml) for 30 minutes to remove 4-methyltrityl group from the side chain of Lys at 26 position. The resultant resin was neutralized with a mixture solution of 5% DIPEA/methylene chloride, washed with methylene chloride and swelled with NMP (30ml). Then, the swelled resin was reacted with palmitoyl-Glu(α-OtBu) (441mg, 1mmol) in the presence of HOBt (135mg, 1mmol) and DCC (260mg, 1mmol) for 3 hours. The obtained peptide resin was treated with 20% piperidine to remove Fmoc, and further treated with a mixture solution of 88% TFA/2% TIPS/ 5% water/5% phenol (20ml) for 1 hour. The resin was filtrated off and the resin was washed with 10ml of TFA, and the combined filtrate was concentrated. The residue was treated with ether to obtain the precipitate (720mg). 500mg of the obtained crude peptide was dissolved in saturated urea solution (200ml) and purified by reversed phase HPLC using C4 (YMC-pack, PROTEIN-RP, 2cm x 25cm) with liner gradient from 13% to 60% acetonitrile/0.1% TFA at flow rate of 5 to 10ml/min. The fraction containing the object product was collected and lyophilized to yield 145mg of the purpose peptide. ESI-MS: 3751 (theoretical value: 3751.2). Leu standard amino acid composition after hydrolysis with 6N hydrochloric acid: Asx: 0.97 (1), Thr: 1.89 (2), Ser: 2.75 (3), Glx: 5.08 (5), Gly: 4.05 (4), Ala: 4.01 (4), Val: 1.93 (2), Ile: 0.99 (1), Leu: 2, Tyr: 0.91 (1), Phe: 1.90 (2), Lys: 1.10 (1), His: 0.90 (1), Arg: 1.92 (2).

### Reference Example 6: Synthesis of GLP-1(7-37)

H-His(Trt)-Ala-Glu(OtBu)-Gly-Thr(Trt)-Phe-Thr(Trt)-Ser(tBu) -Asp(OtBu)-Val-Ser(tBu)-Ser(tBu)-Tyr(tBu)-Leu-Glu(OtBu)-Gly-Gln (Trt)-Ala-Ala-Lys(Boc)-Glu(OtBu)-Phe-Ile-Ala-Trp-Leu-Val-Lys(Boc) -Gly-Arg(Pmc)-Wang resin (1.1g) was constructed from Fmoc-Arg (Pmc)-Wang resin as the starting material by Fmoc method (0.25mmol protocol) using the automatic peptide synthesizer 433A (ABI Co., Ltd.). 600mg of the obtained resin was treated with a mixture solution of 88% TFA/2% TIPS/ 5% water/5% phenol (20ml) for 1 hour. The resin was filtrated off and the filtrate was concentrated and the residue was treated with ether to obtain the precipitate (380mg). The obtained crude peptide was dissolved in purified water (20ml) and purified by reversed phase HPLC using C4 (YMC-pack, PROTEIN-RP, 2cm x 25cm) with liner gradient from 9% to 68% acetonitrile/0.1% TFA at flow rate of 10ml/min. The fraction containing the object product was collected and lyophilized to yield 53mg of the purposed peptide. ESI-MS: [M] 3355 (theoretical value: 3355.7). Leu standard amino acid composition after hydrolysis with 6N hydrochloric acid: Asx: 1.00 (1), Thr: 1.99 (2), Ser: 2.79 (3), Glx: 4.10 (4), Gly: 4.01 (4), Ala: 4.02 (4), Val: 1.92 (2), Ile: 0.98 (1), Leu: 2, Tyr: 0.92 (1), Phe: 1.92 (2), Lys: 2.18 (2), His: 0.96 (1), Arg: 0.94 (1).

### Reference Example 7: Synthesis of [Val⁸]GLP-1(7-37)

H-His(Trt)-Val-Glu(OtBu)-Gly-Thr(Trt)-Phe-Thr(Trt)-Ser(tBu) -Asp(OtBu)-Val-Ser(tBu)-Ser(tBu)-Tyr(tBu)-Leu-Glu(OtBu)-Gly-Gln (Trt)-Ala-Ala-Lys(Boc)-Glu(OtBu)-Phe-Ile-Ala-Trp-Leu-Val-Lys(Boc) -Gly-Arg(Pmc)-Wang resin (1.4g) was constructed from Fmoc-Arg (Pmc)-Wang resin as the starting material by Fmoc method (0.25mmol protocol) using the automatic peptide synthesizer 433A (ABI Co., Ltd.). 0.7g of the obtained resin was treated with a mixture solution of 88% TFA/2% TIPS/ 5% water/5% phenol (20ml) for 1 hour. The resin was filtrated off and the filtrate was concentrated and the residue was treated with ether to obtain the precipitate (327mg). The obtained crude peptide was dissolved in purified water (50ml) and purified by reversed phase HPLC using C4 (YMC-pack, PROTEIN-RP, 2. 5cm x 30cm) with liner gradient from 5% to 72% acetonitrile/0.1% TFA at flow rate of 10ml/min. The fraction containing the object product was collected and lyophilized to yield 75mg of the purposed peptide. ESI-MS: 3383 (theoretical value: 3383.8). Leu standard amino acid composition after hydrolysis with 6N hydrochloric acid: Asx: 0.99 (1), Thr: 1.98 (2), Set: 2.80 (3), Glx: 4.10 (4), Gly: 4.01 (4), Ala: 3.03 (3), Val: 2.86 (3), Ile: 0.98 (1), Leu: 2, Tyr: 0.92 (1), Phe: 1.92 (2), Lys: 2.18 (2), His: 0.92 (1), Arg: 0.94 (1).

### Reference Example 8: Synthesis of Exendin-4

H-His(Trt)-Gly-Glu(OtBu)-Gly-Thr(Trt)-Phe-Thr(Trt)-Ser(tBu)-Asp(OtBu)-Leu-Ser(tBu)-Lys(Boc)-Gln(Trt)-Met-Glu((OtBu)-Glu(OtBu)-Ala-Val-Arg(Pmc)-Leu-Phe-Ile-Glu(OtBu)-Trp-Lue-Lys(Boc)-Asn(Trt)-Gly-Gly-Pro-Ser(tBu)-Ser(tBu)-Gly-Ala-Pro-Pro-Pro-Pro-Ser(tBu)-Wang resin (1.9g) was constructed from Fmoc-Ser(tBu)-Wang resin as the starting material by Fmoc method (0.25mmol protocol). 1. 9g of the obtained resin was treated with a mixture solution of 88% TFA/2% TIPS/5% water/5% phenol (20ml) for 1 hour. The resin was filtrated off and the filtrate was concentrated and the residue was treated with ether to obtain the precipitate (870mg). The obtained crude peptide was dissolved in purified water (80ml) and purified by reversed phase HPLC using C4 (YMC-pack, PROTEIN-RP, 2.5cm x 30cm) with liner gradient from 18 to 72% acetonitrile/0.1% TFA at flow rate of 10ml/min for 60 minutes. The fraction containing the object product was collected and lyophilized to yield 270mg of the purposed peptide. ESI-MS: 4186 (theoretical value: 4186.6). Leu standard amino acid composition after hydrolysis with 6N hydrochloric acid: Asx: 1.99 (2), Thr: 1.97 (2), Ser: 4.77 (5), Glx: 6.06 (6), Gly: 4.97 (5), Ala: 2.02 (2), Val: 0.94 (1), Met: 0.68 (1), Ile: 0.98 (1), Leu: 3, Phe: 1.90 (2), Lys: 2.17 (2), His: 0.98 (1), Arg: 0.92 (1), Pro: 3.96 (4).

### Preparation Example 28 Preparation of pharmaceutical composition for nasal absorption of GLP-1(7-37) containing corn starch (5%) and benzalkonium chloride (0.01%)

About 269mg of calcium carbonate (average particle size: 53. 6µm) was mixed with about 15mg of corn starch (average particle size: 13.3µm) and the mixture was thoroughly mixed. Then, purified water was added to the mixture and the mixture was kneaded. After kneading, the mixture was dried overnight in a freeze-dryer under reduced pressure and passed through 180µm sieve to obtain the dried products. As the peptide component, an amount (about 10.8mg) of the GLP-1 (7-37) powder equivalent to 9. 03mg of GLP-1 (7-37) was mixed with the dried products obtained above. After thoroughly mixing the mixture, a solution containing 0. 03mg of benzalkonium chloride was added and then purified water was further added and the resultant mixture was kneaded. After kneading, the mixture was dried overnight in a desiccator under reduced pressure and the dried products were passed through 180µm sieve. To the sieved mixture, an amount (about 2.2mg) of calcium stearate equivalent to 1.0% of the total weight was admixed to give about 252mg of a powder sample. The GLP-1(7-37) content in the powder sample was measured by a reversed-phase HPLC, and 3mg of the powder sample containing 107µg of GLP-1 (7-37) was prepared as a pharmaceutical composition for nasal absorption.

### Preparation Example 29: Preparation of pharmaceutical composition for nasal absorption. of GLP-1(7-37) containing benzalkonium chloride (0.01%)

As the peptide component, an amount (about 10.5mg) of the GLP-1(7-37) powder equivalent to 8.75mg of GLP-1(7-37) was mixed gradually with about 284mg of calcium carbonate (average particle size: 53.6µm). After thoroughly mixing the mixture, a solution containing 0. 03mg of benzalkonium chloride was added and then purified water was further added and the resultant mixture was kneaded. After kneading, the mixture was dried overnight in a desiccator under reduced pressure and the dried products were passed through 180µm sieve. To the sieved mixture, an amount (about 2.9mg) of calcium stearate equivalent to 1.0% of the total weight was admixed to give about 258mg of a powder sample. The GLP-1(7-37) content in the powder sample was measured by a reversed-phase HPLC, and 3mg of the powder sample containing 102µg of GLP-1 (7-37) was prepared as a pharmaceutical composition for nasal absorption.

### Preparation Example 30: Preparation of pharmaceutical composition for nasal absorption of [Val⁸]-GLP-1(7-37) containing corn starch (5.0%) and benzalkonium chloride (0.01%)

About 269mg of calcium carbonate (average particle size: 53.6µm) was mixed with about 15mg of corn starch (average particle size: 13.3µm) and the mixture was thoroughly mixed. Then, purified water was added to the mixture and the mixture was kneaded. After kneading, the mixture was dried overnight in a freeze-dryer under reduced pressure and passed through 180µm sieve to obtain the dried products. As the peptide component, an amount (about 13mg) of the [Val⁸]-GLP-1(7-37) powder equivalent to 9.38mg of [Val⁸]-GLP-1(7-37) was mixed with the dried products obtained above. After thoroughly mixing the mixture, a solution containing 0.03mg of benzalkonium chloride was added and then purified water was further added and the resultant mixture was kneaded. After kneading, the mixture was dried overnight in a desiccator under reduced pressure and the dried products were passed through 180µm sieve. To the sieved mixture, an amount (about 3.1mg) of calcium stearate equivalent to 1.0% of the total weight was admixed to give about 261mg of a powder sample. The [Val⁸]-GLP-1(7-37) content in the powder sample was measured by a reversed-phase HPLC, and 3mg of the powder sample containing 108µg of [Val⁸]-GLP-1(7-37) was prepared as a pharmaceutical composition for nasal absorption.

### Preparation Example 31: Preparation of pharmaceutical composition for nasal absorption of [Val⁸]-GLP-1(7-37) containing benzalkonium chloride (0.01%)

As the peptide component, an amount (about 13mg) of the [Val⁸]-GLP-1(7-37) powder equivalent to 9.31mg of [Val⁸]-GLP-1(7-37) was mixed gradually with about 284mg of calcium carbonate (average particle size: 53.6µm). After thoroughly mixing the mixture, a solution containing 0. 03mg of benzalkonium chloride was added and then purified water was further added and the resultant mixture was kneaded. After kneading, the mixture was dried overnight in a desiccator under reduced pressure and the dried products were passed through 180µm sieve. To the sieved mixture, an amount (about 2.6mg) of calcium stearate equivalent to 1.0% of the total weight was admixed to give about 252mg of a powder sample. The [Vla⁸]-GLP-1(7-37) content in the powder sample was measured by a reversed-phase HPLC, and 3mg of the powder sample containing 111µg of [Val⁸]-GLP-1(7-37) was prepared as a pharmaceutical composition for nasal absorption.

### Preparation Example 32: Preparation of pharmaceutical composition for nasal absorption of Exendin-4 containing corn starch (5.0%) and benzalkonium chloride (0.01%)

About 270mg of calcium carbonate (average particle size: 53.6µm) was mixed with about 15mg of corn starch (average particle size: 13.3µm) and the mixture was thoroughly mixed. Then, purified water was added to the mixture and the mixture was kneaded. After kneading, the mixture was dried overnight in a freeze-dryer under reduced pressure and passed through 180µm sieve to obtain the dried products. As the peptide component, an amount (about 12mg) of exendin-4 powder equivalent to 9.08mg of exendin-4 was mixed with the dried products obtained above. After thoroughly mixing the mixture, a solution containing 0. 03mg of benzalkonium chloride was added and then purified water was further added and the resultant mixture was kneaded. After kneading, the mixture was dried overnight in a desiccator under reduced pressure and the dried products were passed through 180µm sieve. To the sieved mixture, an amount (about 3.0mg) of calcium stearate equivalent to 1.0% of the total weight was admixed to give about 255mg of a powder sample. The exendin-4 content in the powder sample was measured by a reversed-phase HPLC, and 3mg of the powder sample containing 107µg of exendin-4 was prepared as a pharmaceutical composition for nasal absorption.

### Preparation Example 33: Preparation of pharmaceutical composition for nasal absorption of Exendin-4 containing benzalkonium chloride (0.01%)

As the peptide component, an amount (about 12mg) of the Amylin: exendin-4 powder equivalent to 9.0mg of exendin-4 was mixed gradually with about 285mg of calcium carbonate (average particle size: 53. 6µm). After thoroughly mixing the mixture, a solution containing 0. 03mg of benzalkonium chloride was added and then purified water was further added and the resultant mixture was kneaded. After kneading, the mixture was dried overnight in a desiccator under reduced pressure and the dried products were passed through 180µm sieve. To the sieved mixture, an amount (about 2.6mg) of calcium stearate equivalent to 1.0% of the total weight was admixed to give about 260mg of a powder sample. The exendin-4 content in the powder sample was measured by a reversed-phase HPLC, and 3mg of the powder sample containing 104µg of exendin-4 was prepared as a pharmaceutical composition for nasal absorption.

### Preparation Example 34: Preparation of pharmaceutical composition for nasal absorption of [Lys²⁶, ε-NH{γ-Glu(N-α-palmitoyl)}]-GLP-1(7-37) containing corn starch (5.0%) and benzalkonium chloride (0.01%)

About 270mg of calcium carbonate (average particle size: 53.6µm) was mixed with about 15mg of corn starch (average particle size: 13.3µm) and the mixture was thoroughly mixed. Then, purified water was added to the mixture and the mixture was kneaded. After kneading, the mixture was dried overnight in a freeze-dryer under reduced pressure and passed through 180µm sieve to obtain the dried products. As the peptide component, an amount (about 12mg) of [Lys²⁶, ε-NH{γ-Glu(N-α-palmitoyl)})-GLP-1(7-37) powder equivalent to 9.23mg of [Lys²⁶, ε-NH{γ-Glu(N-α-palmitoyl)}]-GLP-1(7-37) was mixed with the dried products obtained above. After thoroughly mixing the mixture, a solution containing 0. 03mg of benzalkonium chloride was added and then purified water was further added and the resultant mixture was kneaded. After kneading, the mixture was dried overnight in a desiccator under reduced pressure and the dried products were passed through 180µm sieve. To the sieved mixture, an amount (about 2.7mg) of calcium stearate equivalent to 1.0% of the total weight was admixed to give about 245mg of a powder sample. The [Lys²⁶, ε-NH{γ-Glu(N-α-palmitoyl)}]-GLP-1 (7-37) content in the powder sample was measured by a reversed-phase HPLC, and 3mg of the powder sample containing 113µg of [Lys²⁶, ε-NH{γ-Glu(N-α-palmitoyl)}]-GLP-1(7-37) was prepared as a pharmaceutical composition for nasal absorption.

### Preparation Example 35: Preparation of pharmaceutical composition for nasal absorption of [Lys²⁶, ε-NH{γ-Glu(N-α-palmitoyl)}]-GLP-1(7-37) containing benzalkonium chloride (0.01%)

As the peptide component, an amount (about 12mg) of the [Lys²⁶, ε-NH{γ-Glu(N-α-palmitoyl)}]-GLP-1(7-37) powder equivalent to 9.08mg of [Lys²⁶, ε-NH{γ-Glu(N-α-palmitoyl)}]-GLP-1(7-37) was mixed gradually with about 285mg of calcium carbonate (average particle size: 53.6µm). After thoroughly mixing the mixture, a solution containing 0.03mg of benzalkonium chloride was added and then purified water was further added and the mixture was kneaded. After kneading, the mixture was dried overnight in a desiccator under reduced pressure and the dried products were passed through 180µm sieve. To the sieved mixture, an amount (about 2.4mg) of calcium stearate equivalent to 1.0% of the total weight was admixed to give about 265mg of a powder sample. The [Lys²⁶, ε-NH{γ-Glu(N-α-palmitoyl)}]-GLP-1(7-37) content in the powder sample was measured by a reversed-phase HPLC, and 3mg of the powder sample containing 103µg of [Lys²⁶, ε-NH{γ-Glu(N-α-palmitoyl)}]-GLP-1(7-37) was prepared as a pharmaceutical composition for nasal absorption.

### Example 5: The promoting effect of starch as additive for nasal absorption of acidic polypeptides

This example shows the absorption enhancement effect of starch addition for nasal absorption of human insulin using cynomolgus monkeys. Human insulin is used as one of the examples of acidic polypeptides other than incretin hormone.

Fine powder of either calcium carbonate or sulfated aluminum salt of sucrose (sucralfate) with an average particle size of 50µm was used as the carrier. According to the procedures described in Preparation Examples 10 to 18, pharmaceutical compositions for nasal absorption containing Domyo-ji powder (1.0%) were prepared. The dosage of human insulin was adjusted so that the about 25IU (International Unit)/monkey/40mg of the composition was administered. Two additive-free formulations, one containing calcium carbonate and human insulin and the other containing sucralfate and human insulin, were prepared to serve as controls.

Using a nasal nebulizer manufactured by UNISIA JECS Co., Ltd., about 40mg of the compositions of Preparation Examples 36 to 38 were individually administered to cynomolgus monkeys, each weighing about 3kg, in their nasal cavities. The concentrations of insulin and glucose in the plasma were measured by insulin - RIA beads II (Dinabott Co., Ltd.) and the measurement instrument for blood glucose level (FREESTYLE-KISSEI; KISSEI Pharmaceutical Co., Ltd.) at 0, 5, 15, 20, 30, 45, 60, 90, and 120 minutes after administration.

The results are shown in Tables 9 and 10. As can be seen from the results, the absorption of human insulin and decreasing of the blood glucose level were observed by administration of the additive-free composition. However, in the case of the administration of the composition containing 1.0% of Domyo-ji powder (partially pregelatinized starch), increase in the absorbability of human insulin was observed and the significantly low blood glucose level was observed in the early stage.

**Table 9:**

| Plasma concentration of human insulin by administration of the composition containing human insulin | | | | | | |
|---|---|---|---|---|---|---|
| Composition | Time after administration (min) | | | | | |
| | Before | 5 | 10 | 15 | 60 | 120 |
| Calcium carbonate | 20.5 | 56.0 | 166.5 | 297.0 | 47.0 | 16.0 |
| Calcium carbonate/Domyo-ji powder (1%) | 12.0 | 115.5 | 267.5 | 275.0 | 56.5 | 41.5 |
| Sucralfate | 15.0 | 72.5 | 183.0 | 185.0 | 44.0 | 17.5 |

**Table 10:**

| Blood glucose level by administration of the composition containing human insulin | | | | | | |
|---|---|---|---|---|---|---|
| Composition | Time after administration (min) | | | | | |
| | Before | 5 | 10 | 15 | 60 | 120 |
| Calcium carbonate | 60.0 | 88.0 | 96.5 | 78.5 | N.D. | N.D. |
| Calcium carbonate/Domyo-ji powder (1%) | 55.0 | 82.5 | 73.5 | 33.5 | N.D. | N.D. |
| Sucralfate | 72.5 | 74.0 | 45.0 | 77.0 | 20.0 | 21.5 |
| N.D.: not detected | | | | | | |

### Preparation Example 36: Preparation of pharmaceutical composition of insulin/sucralfate

To 120mg of sucralfate was added 1ml of human insulin preparation (Humulin R: Shionogi Pharmaceutical Co.,' Ltd.) and the mixture was thoroughly mixed. Then, the mixture was dried overnight under reduced pressure and the dried products were passed through 180µm sieve. To the sieved mixture, an amount (about 1.2mg) of calcium stearate equivalent to 1.0% of the total weight was admixed to give a powder sample. 40mg of the powdered sample containing about 25IU of human insulin was filled in #2 capsules to prepare a pharmaceutical composition for nasal absorption.

### Preparation Example 37: Preparation of pharmaceutical composition of insulin/calcium carbonate

To 120mg of calcium carbonate (mean particle size: 53.6µm) was added 1ml of human insulin preparation (Humulin R: Shionogi Pharmaceutical Co., Ltd.) and the mixture was thoroughly mixed. Then, the mixture was dried overnight under reduced pressure and the dried products were passed through 180µm sieve. To the sieved mixture, an amount (about 1.2mg) of calcium stearate equivalent to 1.0% of the total weight was admixed to give a powder sample. 40mg of the powdered sample containing about 25IU of human insulin was filled in #2 capsules to prepare a pharmaceutical composition for nasal absorption.

### Preparation Example 38: Preparation of pharmaceutical composition of insulin/calcium carbonate containing Domyo-ji powder (1.0%)

To 120mg of calcium carbonate (mean particle size: 53.6µm) was added a suspension of 1ml of human insulin preparation (Humulin R: Shionogi Pharmaceutical Co., Ltd.) and 1.2mg of Domyo-ji powder and the mixture was kneaded. After kneading, the mixture was dried overnight under reduced pressure and the dried products were passed through 180µm sieve. To the sieved mixture, an amount (about 1.2mg) of calcium stearate equivalent to 1.0% of the total weight was admixed to give a powder sample. 40mg of the powdered sample containing about 25IU of human insulin was filled in #2 capsules to prepare a pharmaceutical composition for nasal absorption.

### Example 6: Affect of a particle size of starch as an additive of a pharmaceutical composition on nasal absorption

This example examined the effect of the particle size of starch as the additive of the pharmaceutical composition containing GLP-1(7-36)NH₂ on nasal absorption using decreasing activity of blood glucose level in rats as an index.

The following two different fine powder starches with an average particle size were used as the additives of the pharmaceutical composition of this example.

### Corn starch:

Average of integrated particle size (d50): 13.3µm
Central distribution: 14µm
Distribution range: 5.5µm to 30µm

### Potato starch:

Average of integrated particle size (d50): 37.7µm
Central distribution: 45µm
Distribution range: 5.5µm to 105µm

CD (SD) rats weighing about 300g were anaesthetized with pentbarbital. The front edge of tip for pipette (GPS-250, RAININ) was filled with 3mg of a pharmaceutical composition (containing about 90µg of polypeptide) prepared by the Preparation Example described later, and the tip was charged in the syringe, and then the composition was sprayed in the nasal cavity of the rats with 1ml of air. Then, 0.5g/kg of glucose was intravenously administered in the tail of the rats 5 minutes after nasal administration of the composition. The blood samples were collected from the aorta 15 minutes after nasal administration of the composition (10 minutes after glucose administration). The blood glucose level was detected using the measurement instrument for blood glucose level (FREESTYLE-KISSEI; KISSEI Pharmaceutical Co., Ltd.)

The results are shown in Table 11 below. As can be seen from the results, the blood glucose level of the control group without administration of GLP-1(7-36)NH₂ was 196mg/dl (average value three rats). The blood glucose level of the groups administered with the composition containing GLP-1(7-36)NH₂ without the additive, with 5% corn starch and 5% potato starch were 170mg/dl, 157mg/dl and 182mg/dl, respectively. These blood glucose levels were lower than that of the control group without administration of GLP-1(7-36)NH₂.

The absorption enhancement effect for absorption of GLP-1(7-36)NH₂ was preferably observed in the case of the group using corn starch with small particle size in comparison with the one using potato starch with large particle size.

As apparent from the results of table 11, preferable nasal absorption of GLP-1(7-36)NH₂ can be seen when using carrier with smaller particle size, whereas the larger particle sized starch dose not show clear absorption enhancement effect.

**Table 11:**

| Affect of a particle size of starch on the activity to decrease the blood glucose level by GLP-1(7-36)NH₂ | | | |
|---|---|---|---|
| Peptide | Composition | Average value | SD |
| Control | - | 196 | 12 |
| GLP-1(7-36)NH₂ | No additive | 170 | 0 |
| | 5% corn starch (13.3µm) | 157 | 8 |
| | 5% potato starch (37.7µm) | 182 | 15 |

### Preparation Example 39: Preparation of additive-free pharmaceutical composition

As the peptide component, an amount (about 108mg) of the GLP-1(7-36)NH₂ powder equivalent to 90.2mg of GLP-1(7-36)NH₂ was mixed with about 2.86g of calcium carbonate. After thoroughly mixing the mixture, a solution containing 0. 3mg of benzalkonium chloride was added and then purified water was further added and the mixture was kneaded. After kneading, the mixture was dried overnight in a freeze-dryer under reduced pressure and the dried products were passed through 180µm meshed sieve. To the sieved mixture, an amount (about 30mg) of calcium stearate equivalent to 1.00 of the total weight was admixed to give 2.95g of a powder sample. The GLP-1(7-36)NH₂ content in the powder sample was measured by a reversed-phase HPLC, and 3mg of the powder sample containing 92µg of GLP-1(7-36)NH₂ was prepared as a pharmaceutical composition for nasal absorption.

### Preparation Example 40: Preparation of pharmaceutical composition containing corn starch (5.0%)

To about 2.71g of calcium carbonate (average particle size: 53.6µm) were added about 150mg of corn starch (average particle size: 13.3µm) and the mixture was thoroughly mixed, and then purified water was added and the mixture was kneaded. After kneading, the mixture was dried overnight in a freeze-dryer under reduced pressure and passed through a 180µm sieve to obtain the dried products. As the peptide component, an amount (about 107mg) of the GLP-1(7-36)NH₂ powder equivalent to 89.2mg of GLP-1(7-36)NH₂ was mixed with the dried products obtained above. After thoroughly mixing the mixture, a solution containing 0.3mg of benzalkonium chloride was added and then purified water was further added and the mixture was kneaded. After kneading, the mixture was dried overnight in a freeze-dryer under reduced pressure and the dried products were passed through 180µm sieve. To the sieved mixture, an amount (about 29mg) of calcium stearate equivalent to 1.0% of the total weight was admixed to give about 2. 90g of a powder sample. The GLP-1(7-36)NH₂ content in the powder sample was measured by a reversed-phase HPLC, and 3mg of the powder sample containing 92µg of GLP-1(7-36)NH₂ was prepared as a pharmaceutical composition for nasal absorption.

### Preparation Example 41: Preparation of pharmaceutical composition containing potato starch (5.0%)

To about 2.71g of calcium carbonate (average particle size: 53.6µm) were added about 150mg of potato starch (average particle size: 37.7µm) and the mixture was thoroughly mixed, and then purified water was added and the mixture was kneaded. After kneading, the mixture was dried overnight in a freeze-dryer under reduced pressure and passed through a 180µm sieve to obtain the dried products. As the peptide component, an amount (about 107mg) of the GLP-1(7-36)NH₂ powder equivalent to 89.5mg of GLP-1(7-36)NH₂ was mixed with the dried products obtained above. After thoroughly mixing the mixture, a solution containing 0. 3mg of benzalkonium chloride was added and then purified water was further added and the mixture was kneaded. After kneading, the mixture was dried overnight in a freeze-dryer under reduced pressure and the dried products were passed through 180µm sieve. To the sieved mixture, an amount (about 29mg) of calcium stearate equivalent to 1.0% of the total weight was admixed to give about 2.88g of a powder sample. The GLP-1(7-36)NH₂ content in the powder sample was measured by a reversed-phase HPLC, and 3mg of the powder sample containing 93µg of GLP-1(7-36)NH₂ was prepared as a pharmaceutical composition for nasal absorption.

### INDUSTRIAL APPLICABILITY

As set forth, the present invention provides a novel pharmaceutical composition for nasal absorption, which contains a biologically active polypeptide, a carrier that is insoluble or little soluble in water, and an additive capable of uniformly dispersing and embedding the polypeptide on the surfaces of the carrier. The unprecedented pharmaceutical composition improves the absorbability of biologically active polypeptides, in particular, the acidic biologically active polypeptides having an isoelectric point of 7 or lower and, thus, a low solution stability, that are otherwise difficult to administer via any other non-injection administration routes including oral administration.

Accordingly, the pharmaceutical composition of the present invention for nasal absorption enables nasal administration, or application to nasal mucosa, of insulin secretion-promoting polypeptides, for which viable non-injection administration route has yet to be established, in the form of a powder composition. In this manner, the composition enhances the bioavailability of the polypeptide and can thus serve as a clinically effective medicament. For this reason, the pharmaceutical composition of the present invention has a significant medical impact.

## Claims

1. A pharmaceutical composition for nasal absorption, comprising a biologically active acidic polypeptide with an isoelectric point of 7 or lower, a carrier that is insoluble or little soluble in water, and an additive for dispersing and embedding the polypeptide on the surface of the carrier.

2. A pharmaceutical composition for nasal absorption claimed in claim 1, comprising a biologically active acidic polypeptide with an isoelectric point of 7 or lower, a carrier that is insoluble or little soluble in water, and an additive for dispersing and embedding the polypeptide on the surface of the carrier with the average particle size of 1µm to 20µm.

3. A pharmaceutical composition for nasal absorption claimed in claim 1 or 2, comprising a biologically active acidic polypeptide with an isoelectric point of 7 or lower, a carrier that is insoluble or little soluble in water, and an additive for dispersing and embedding the polypeptide on the surface of the carrier that is insoluble or little soluble in water, with the average particle size of 1µm to 20µm.

4. The pharmaceutical composition for nasal absorption according to claim 1, 2 or 3, wherein the carrier that is insoluble or little soluble in water is a polyvalent metal compound.

5. The pharmaceutical composition for nasal absorption according to claim 4, wherein the polyvalent metal compound is an aluminum compound, a calcium compound, a magnesium compound, a silicon compound, an iron compound, or a zinc compound.

6. The pharmaceutical composition for nasal absorption according to claim 5, wherein the aluminum compound is one selected from the group consisting of dried aluminum hydroxide gel, chlorohydroxy aluminum, synthetic aluminum silicate, light aluminum oxide, colloidal hydrous aluminum silicate, aluminum magnesium hydroxide, aluminum hydroxide, aluminum hydroxide gel, aluminum sulfate, dihydroxy aluminum acetate, aluminum stearate, natural aluminum silicate, aluminum monostearate, and aluminum potassium sulfate.

7. The pharmaceutical composition for nasal absorption according to claim 5, wherein the calcium compound is one selected from the group consisting of apatite, hydroxyapatite, calcium carbonate, calcium disodium edetate, calcium chloride, calcium citrate, calcium glycerophosphate, calcium gluconate, calcium silicate, calcium oxide, calcium hydroxide, calcium stearate, calcium tertiary phosphate, calcium lactate, calcium pantothenate, calcium oleate, calcium palmitate, D-calcium pantothenate, calcium alginate, anhydrous calcium phosphate, calcium hydrogen phosphate, calcium dihydrogen phosphate, calcium acetate, calcium saccharate, calcium sulfate, calcium monohydrogen phosphate, calcium para-aminosalicylate, and biologically calcified compounds.

8. The pharmaceutical composition for nasal absorption according to claim 5, wherein the magnesium compound is one selected from the group consisting of magnesium L-aspartate, magnesium chloride, magnesium gluconate, magnesium aluminosilicate, magnesium silicate, magnesium oxide, magnesium hydroxide, magnesium stearate, magnesium carbonate, magnesium aluminometasilicate, magnesium sulfate, magnesium sodium silicate, and synthetic magnesium sodium silicate.

9. The pharmaceutical composition for nasal absorption according to claim 5, wherein the silicon compound is one selected from the group consisting of hydrous silicon dioxide, light anhydrous silicic acid, synthetic hydrotalcite, diatomite, and silicon dioxide.

10. The pharmaceutical composition for nasal absorption according to claim 5, wherein the iron compound is iron sulfate.

11. The pharmaceutical composition for nasal absorption according to claim 5, wherein the zinc compound is one selected from the group consisting of zinc chloride, zinc stearate, zinc oxide, and zinc sulfate.

12. The pharmaceutical composition for nasal absorption according to any one of claims 4 to 11 wherein the polyvalent metal compound has an average particle size of 100µm or less.

13. The pharmaceutical composition for nasal absorption according to claim 12, wherein the polyvalent metal compound has an average particle diameter of 20 to 60µm.

14. The pharmaceutical composition for nasal absorption according to any one of claims 1 to 13, wherein the biologically active acidic polypeptide is one selected from the group consisting of calcitonin, katacalcin, cholecystokinin-12, cholecystokinin-8, corticotropin-lipotropin precursor, corticotropin-like intermediate peptide, lipotropin-β, lipotropin-γ, melanotropin-β, corticoliberin, endothelin-1, endothelin-2, endothelin-3, galanin message-associated peptide, gastrin-71, gastrin-34, gastrin-17, gastric inhibitory polypeptide, glicentin-related polypeptide, glucagon, glucagon-like peptide-1, glucagon-like peptide-1 amide, glucagon-like peptide-1(7-36) amide, glucagon-like peptide-1(7-37), [Val⁸]-glucagon-like peptide-1(7-36) amide, [Val⁸]-glucagon-like peptide-1(7-37), [Lys²⁶, ε-NH{γ-Glu(N-α-palmitoyl)}]-GLP-1(7-37), glucagon-like peptide-1(9-36) amide, glucagon-like peptide-1(9-37), glucagon-like peptide-2, exendin-3, exendin-4, insulin β-chain, insulin α-chain, insulin, progonadoliberin-I, gonadoliberin-II, gonadoliberin-related peptide-I, neuromedin C, insulin-like protein (INSL) A-chain, motilin-related peptide E, leucine-enkephalin, methionine-enkephalin, leumorphin, oxytocin, neurophysine-1, neurophysine-2, copeptin, neuromedin B, neuromedin N, neuropeptide Y, neuropeptide AF, PACAP-related peptide, pancreatic hormone, pancreatic icosapeptide, peptide YY, tyroliberin, neuroquinine A, urocortin, urotensin II, intestinal peptide (PHM-27), and intestinal peptide-42.

15. The pharmaceutical composition for nasal absorption according to claim 14, wherein the biologically active acidic polypeptide is one selected from the group consisting of glucagon-like peptide-1, glucagon-like peptide-1 amide, glucagon-like peptide-1(7-36) amide, glucagon-like peptide-1(7-37), [Val⁸]-glucagon-like peptide-1(7-36) amide, [Val⁸]-glucagon-like peptide-1(7-37), [Lys²⁶, ε-NH{γ-Glu(N-α-palmitoyl)}]-GLP-1(7-37), glucagon-like peptide-1(9-36) amide, glucagon-like peptide-1(9-37), glucagon-like peptide-2, exendin-3, exendin-4, glucagon, gastric inhibitory polypeptide, insulin and derivatives thereof.

16. The pharmaceutical compound for nasal absorption according to any one of claims 1 to 13, comprising peptide incretin, a carrier that is insoluble or little soluble in water, and an additive for dispersing and embedding the peptide incretin on the surface of the carrier.

17. The pharmaceutical compound for nasal absorption according to claim 16, wherein peptide incretin is one selected from the group consisting of glucagon-like peptide-1, glucagon-like peptide-1 amide, glucagon-like peptide-1(7-36) amide, glucagon-like peptide-1(7-37), [Val⁸]-glucagon-like peptide-1(7-36) amide, [Val⁸]-glucagon-like peptide-1(7-37), [Lys²⁶, ε-NH{γ-Glu(N-α-palmitoyl)}]-GLP-1(7-37), glucagon-like peptide-1(9-36) amide, glucagon-like peptide-1(9-37), glucagon-like peptide-2, exendin-3, exendin-4, glucagon, gastric inhibitory polypeptide, insulin and derivatives thereof.

18. The pharmaceutical compound for nasal absorption according to any one of claims 1 to 17, wherein the additive is a starch selected from the group consisting of amylopectin, amylose, or a mixture containing amylopectin and amylose at any proportion.

19. The pharmaceutical compound for nasal absorption according to any one of claims 1 to18, wherein the additive is rice flour, rice starch, rice beta-starch (nonglutinous rice type), rice beta-starch (glutinous rice type), pregelatinized rice starch (nonglutinous rice type), pregelatinized rice starch (glutinous rice type), corn starch, corn beta-starch (nonglutinous rice type), corn beta-starch (glutinous rice type), pregelatinized corn starch (nonglutinous rice type), pregelatinized corn starch (glutinous rice type), potato starch, potato beta-starch (nonglutinous rice type), pregelatinized potato starch (nonglutinous rice type), pregelatinized wheat starch (nonglutinous rice type), or a partially pregelatinized starch thereof.

20. The pharmaceutical compound for nasal absorption according to any one of claims 1 to 19, wherein the additive is an oligo saccharide, carboxyvinyl polymer, povidone, hydroxypropylcellulose, xanthan gum, pectin, sodium alginate, powdered gum arabic, or gelatin.

21. A pharmaceutical composition containing the pharmaceutical composition for nasal absorption according to any one of claims 1 to 20 along with a DPP-IV inhibitor.

22. The pharmaceutical composition according to claim 21, wherein the DPP-IV inhibitor is diprotin A, bacitracin, or isoleucine thiazolidide.
